(19) **Europäisches Patentamt**
**European Patent Office**
**Office européen des brevets**

(11) **EP 2 889 304 A1**

(12) **EUROPEAN PATENT APPLICATION**

(43) Date of publication:
**01.07.2015 Bulletin 2015/27**

(51) Int Cl.:
***C07K 1/113*** *(2006.01)*

(21) Application number: **13199710.8**

(22) Date of filing: **27.12.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **SANDOZ AG**
**4056 Basel (CH)**

(72) Inventors:
• **Hahn, Rainer**
**1180 Wien (AT)**

• **Pan, Siqi**
**1190 Wien (AT)**
• **Jungbauer, Alois**
**1210 Wien (AT)**
• **Zelger, Monika**
**1170 Wien (AT)**

(74) Representative: **Sonn & Partner Patentanwälte**
**Riemergasse 14**
**A-1010 Wien (AT)**

(54) **Method for refolding recombinantly produced polypeptides**

(57) Disclosed is a method for refolding recombinantly produced polypeptides comprising the steps of
(a) providing inclusion bodies comprising recombinantly produced polypeptides comprising the recombinantly produced polypeptides,
(b) dissolving the inclusion bodies under chaotropic conditions in so as to obtain denatured recombinantly produced polypeptides, and
(c) refolding the denatured recombinantly produced polypeptides inclusion bodies by addition of a refolding buffer, so as to obtain refolded recombinantly produced polypeptides, and
(d) further processing the refolded recombinantly produced polypeptides by at least one processing step,
wherein at least steps (c) and (d) are performed in a tubular reactor in a continuous manner.

EP 2 889 304 A1

**Description**

The invention relates to a method for refolding recombinantly produced polypeptides.

Escherichia coli can produce large amounts of therapeutic proteins with high homogeneity. These proteins are frequently overexpressed and deposited as insoluble aggregates in E. coli, known as inclusion bodies (IBs). It is a very economical way of producing recombinant proteins but refolding is the major cost drive in downstream processing and continuous processing may a way to improve process economics. The advantages of IBs expression include easy isolation by cell disruption and centrifugation, protection from proteolysis and the ability to produce toxic proteins. IBs solubilisation and refolding steps are subsequently performed in chaotropic and kosmotropic buffers respectively for proteins to become biologically active. Usually the refolding step is performed in large refolding tanks in a batch or fed batch mode.

N$^{pro}$ fusion technology represents an efficient E. coli expression system for production of peptides and proteins in the form of IBs as fusion proteins. The system comprises of the autoprotease N$^{pro}$ from classical swine fever virus, which becomes active under refolding conditions, releasing the fused target protein with an authentic N-termini. The variant of N$^{pro}$, EDDIE when fused to a target peptide, showed improved solubility and faster refolding and cleavage (Achmüller et al. 2007, Nature Methods 4(12):1037-1043).

Currently, the simplest and most widely used method in biopharmaceutical manufacturing in protein refolding is the direct dilution method, performed in stirred tanks in a batch wise manner, also called refolding tank. To achieve higher refolding yields, low protein concentrations between 10 to 100$\mu$g/ml is refolded in large stirred tank, presenting itself as a process bottle neck (Jungbauer et al., 2007, J Biotechnol 128(3):587-96). Furthermore large industrial stirred tank have mixing times lasting several minutes. Such steps are also compromising process quality and yield. Large stirred tanks also increases holding times between process steps and is less flexible to any process changes.

Alternative protein refolding reactors were previously developed to circumvent these problems. These include the continuous refolding of $\alpha$-lactalbumin performed in a continuous stirred tank reactor (Schlegl et al., 2005. Science 60(21):5770-5780), a flow type packed column reactor which denatured lysozyme was continuously refolded (Terashima et al., 1996, Process Biochemistry 31(4):341-345), and a membrane tube reactor equipped with paddles and partitioning disks where denatured lysozyme was gradually added from the outer surface of tube into a refolding buffer flowing continuously inside the tube (Katoh et al., 2000, Process Biochemistry 35(10):1119-1124).

It is therefore an object of the present invention to provide an improved method for refolding recombinantly produced polypeptides, especially from inclusion bodies produced in recombinant host cells, such as E. coli. The improvement should allow increased productivity/overall production times, even on an industrial scale and more efficient production process.

Therefore, the present invention provides a method for refolding recombinantly produced polypeptides comprising the steps of

> (a) providing inclusion bodies comprising recombinantly produced polypeptides comprising the recombinantly produced polypeptides,
> (b) dissolving the inclusion bodies under chaotropic conditions in so as to obtain denatured recombinantly produced polypeptides,
> (c) refolding the denatured recombinantly produced polypeptides addition of a refolding buffer, so as to obtain refolded recombinantly produced polypeptides, and
> (d) further processing the refolded recombinantly produced polypeptides by at least one processing step,

wherein at least steps (c) and (d) are performed in a tubular reactor in a continuous manner.

The method according to the present invention provides a continuous refolding and further processing which enables significant advantages especially when performing the present invention on an industrial scale. For example, productivity increases significantly because emptying and filling times are eliminated for the tubular reactor. Preferably, a fully continuous process is provided (so that also step (b) or preferably also steps (a) and (b) are performed in a fully continuous manner).

Step (d) may be any step that is usually foreseen in the batch or semi-continuous processes already known in the present field that further processes the refolded polypeptides to the final product. Examples for such further processing steps are filtration, crystallisation, centrifugation, precipitation, (continuous) chromatography, modification steps (wherein the polypeptide is chemically or biologically (e.g. enzymatically) modified; e.g. cleavage steps (especially autocleavage)), flocculation, heat treatment, pH treatment, etc. or combinations thereof). It is also possible to repeat (once, twice or several times, if necessary) such further processing steps or combination of steps in the tubular reactor. According to the present invention, at least the first further processing step ("step (d)") is performed in a tubular reactor in a continuous manner; however, it is preferred to perform as many steps of the further processing as possible in this manner. It is clear that most further processing steps can either be carried out in the tubular reactor or after discharging from the tubular reactor (but still in continuously processed manner); a few further processing steps (such as centrifugation) are not necessarily suitable for being carried out in a tubular reactor, but can still be carried out continuously (with other unit equipment) in the process according to the present invention (in the case of centrifugation after the product has left the tubular reactor). According to the present invention, at least one further processing step needs to be carried out in the tubular reactor in continuous manner. Preferably, at least two, more preferred at least three, especially at least four, further processing steps are carried out in continuous manner and/or in the tubular reactor. It is, however, also possible to carry out a second, third, fourth, etc. further processing step (such as centrifugation) outside the tubular reactor, but, preferably, still in a continuous manner, e.g. with further unit equipment attached, connected and/or combined with the tubular reactor.

It is specifically preferred to provide a fully continuous process, especially from the beginning (step (a)) to the final processing of the polypeptide of interest (including any further processing step following step (d), which, again are steps, such as filtration, crystallisation, centrifugation, precipitation, (continuous) chromatography, modification steps (wherein the polypeptide is chemically or biologically (e.g. enzymatically) modified; e.g. cleavage steps (especially autocleavage)), etc. flocculation, heat treatment, pH treatment or combinations thereof). The method according to the present invention can be designed as fully integrated reactor applying a completely continuous performance of providing the recombinantly produced polypeptide in purified form. In fact, the present process can even be established as fully continuous process up to bulk product and finished polypeptide.

In the method according to the present invention, at least refolding and at least one further processing step are performed in a tubular reactor. A "tubular reactor" is - according to a suitable definition - a long hollow cylinder for transporting liquid through which flow is continuous, usually at steady state, and configured so that conversion of the chemicals and other dependent variables are functions of position within the reactor rather than of time. In the ideal tubular reactor, the fluids flow as if they were plugs or pistons, and reaction time is the same for all flowing material at any given tube cross section.

In a "continuous process" as meant for the present invention, the reactants are fed into the reactor (or system) and products (e.g. bulk product and/or finished polypeptide) leave the system without accumulation of reactant or product. The continuous process reaches a steady state. Only at the start-up and shut-down such process is unsteady.

The method according to the present invention is performed without the need for a refolding tank, because refolding is performed in the tubular reactor in motion through the tubes and because collecting the recombinantly produced polypeptide (a step that is otherwise mandatory in discontinuous batch or semi-discontinuous batch/flow processing set-ups) is not necessary in the method according to the present invention. This allows significant reduction of the overall processing time.

The method using a tubular reactor ("plug flow reactor model"; "continuous flow reactor"; "piston flow reactor") according to the present invention avoids long mixing times at large scale operations. Using pumps, tubings and various tube connectors, the tubular reactor has the flexibility to be easily constructed into different configurations than other reactors, suiting different protein refolding behaviours and refolding times. The tubings high surface area to volume allows fast heat transfer, thus better temperature control. Disposable tubings can also be used, avoiding cleaning and sterilizing steps. Due to its continuous flow characteristic, it enables bioprocesses to become truly continuous, allowing improved scalability and offers possibility of a direct connection to capture systems such as expanded bed chromatography.

Although flow-type reactors have been used in the past for refolding (Terashima et al., 1996, 341-5; Katoh et al., 2000), the present invention provides a fully continuous process for processing inclusion bodies in an industrially usable scale. The process according to the present invention allows more efficient production of recombinant proteins from recombinantly produced inclusion bodies.

The method according to the present invention provides, in general, continuous bioprocessing that offers higher productivity and a smaller footprint than traditional batch bioprocessing. These continuous strategies can preferably include periodic counter-current chromatography and multicolumn countercurrent solvent gradient purification (Muller-Spath et al., 2010, Biotechnol Bioeng 107(6):974-84). Other advantages of the continuous processes according to the present invention include steady-state operation, small equipment size, high volumetric productivity, streamlined process flow, low cycle times and reduced capital cost.

Preferably, step (c) is performed with changing conditions, especially with changing temperature, with changing ion concentration in the refolding buffer, with changing pH in the refolding buffer, changing protein concentration (e.g. by pulse refolding; see example 2), or combinations or sequential application of such changing conditions. The tubular reactor set-up according to the present invention is specifically advantageous for performing such further processing steps. Also those have been frequently performed in the past by batchwise handling.

Since at least steps (c) and (d) are performed in a continuous manner in the tubular reactor according to the present invention, these steps may also be combined, i.e. step (d) (or any further step) may be performed before solubilisation has been finally or quantitatively completed. This allows a shift to steady state and therefore provides significant processing advantage specifically compared to (discontinuous or semi-continuous) batch process set-ups.

According to a preferred embodiment of the present invention the refolded recombinantly produced polypeptides are in step (d) subjected to a precipitation step, wherein the recombinantly produced polypeptides are obtained in the precipitate. In other embodiments, the product of interest (the recombinantly produced polypeptides are in the supernatant and the impurities are in the precipitate. Suitable precipitating/non-precipitating conditions for almost any recombinant polypeptide can be established based on the known physicochemical (solubility) properties of the polypeptide of interest to be purified and processed according to the present invention.

According to a preferred embodiment, the recombinantly produced polypeptides are fusion polypeptides comprising a polypeptide of interest and an $N^{pro}$ autoprotease.

This "$N^{pro}$ autoprotease technology" using the autoprotease $N^{pro}$ of Pestivirus is specifically useable with the present invention for the recombinant production of a desired heterologous polypeptide of interest. This technology usually provides the recombinant expression of a fusion polypeptide (as a "recombinantly produced polypeptide" according to the present invention) which comprises an autoproteolytic moiety directly or indirectly derived from autoprotease $N^{pro}$ of Pestivirus and a heterologous polypeptide of interest in a host cell, often a prokaryotic host cell, such as E. coli. The heterologous polypeptide or protein of interest is covalently coupled via a peptide bond to the $N^{pro}$ molecule. The protein of interest is released from the fusion protein through hydrolysis of the peptide bond between the C-terminal Cys168 of Npro and position 169 of the fusion polypeptide which represents the authentic N-terminal amino acid of the protein of interest to be produced according to the present invention. The heterologous polypeptide of interest is produced in the host cell in form of cytoplasmic inclusion bodies (IB), which are then isolated and treated in such a way, that the desired heterologous polypeptide is cleaved from the fusion polypeptide by the $N^{pro}$ autoproteolytic activity.

Fusion polypeptides comprising the autoprotease $N^{pro}$ of Pestivirus are therefore specifically useful for producing heterologous recombinant polypeptides. $N^{pro}$ is an autoprotease with length of 168 amino acids and an apparent $M_r$ of about 20 kD in vivo. It is the first protein in the polyprotein of Pestiviruses and undergoes autoproteolytic cleavage from the following nucleocapsid protein C. This cleavage takes place after the last amino acid in the sequence of $N^{pro}$, Cys168. The autoprotease $N^{pro}$ activity of Pestivirus always cleaves off the fusion partner at this clearly determined site, releasing a polypeptide of interest with homogenous N-terminus. In addition, the autoproteolytic activity of $N^{pro}$ can be induced in vitro, by application of special buffers, so that the polypeptide of interest can be obtained by cleavage of fusion polypeptides that are expressed in IBs.

N-terminal autoprotease $N^{pro}$ from Classical Swine Fever Virus (CSFV) used in this technology serves as an attractive tool for expression of therapeutic proteins in large amounts especially in E. coli. Medical applications require an authentic N-terminus of the recombinant proteins, which can be achieved by self-cleavage of N-terminally fused $N^{pro}$ autoprotease. In addition, $N^{pro}$ fusion technology also allows the expression of small or toxic peptides, which would be degraded immediately after their synthesis by host cell proteases (Achmüller et al., Nat. Methods 4 (2007), 1037-1043). As the expression of $N^{pro}$ fusion proteins in E. coli leads to the formation of inclusion bodies, appropriate resolving and renaturation protocols are required to obtain biological active proteins.

The present invention is carried out with the $N^{pro}$ technology. This technology is disclosed e.g. in WO 01/11057 A, WO 01/11056 A, WO 2006/113957 A, WO 2006/113958 A, WO 2006/113959 A, EP 12198006.4, EP 12198007.2 and Achmüller et al., Nat. Meth. 4 (2007), 1037-1043. In general terms, the $N^{pro}$ technology relates to a process for the recombinant production of a heterologous protein of interest, comprising (i) cultivation of a bacterial host cell which is transformed with an expression vector which comprises a nucleic acid molecule which codes for a fusion protein, the fusion protein comprising a first polypeptide which exhibits the autoproteolytic function of an autoprotease $N^{pro}$ of a pestivirus, and a second polypeptide which is connected to the first polypeptide at the C-terminus of the first polypeptide in a manner such that the second polypeptide is capable of being cleaved from the fusion protein by the autoproteolytic activity of the first polypeptide, and the second polypeptide being a heterologous protein of interest, wherein cultivation occurs under conditions which cause expression of the fusion protein and formation of corresponding cytoplasmic inclusion bodies, (ii) isolation of the inclusion bodies from the host cell, (iii) solubilisation of the isolated inclusion bodies, (iv) dilution of the solubilisate to give a reaction solution in which the autoproteolytic cleavage of the heterologous protein of interest from the fusion protein is performed, and (v) isolation of the cleaved heterologous protein of interest ("recovering"). At least steps (iii) and (iv) are carried out in a tubular reactor according to the present invention.

This technology is suited for a large variety of proteins of interest. For the purpose of the present invention, the terms "heterologous protein", "target protein", "polypeptide of interest" or "protein of interest" (and the like) mean a polypeptide which is not naturally cleaved by an autoprotease $N^{pro}$ of a Pestivirus from a naturally occurring fusion protein or polyprotein (i.e. a polypeptide being different than the naturally following amino acids 169ff of the Pestivirus polyprotein encoding the structural Protein C and subsequent viral proteins). Examples of such heterologous proteins of interest are industrial enzymes (process enzymes) or polypeptides with pharmaceutical, in particular human pharmaceutical, activity.

Due to its autocatalytic cleavage it enables synthesis of proteins with an authentic N-terminus which is especially important for pharmaceutical applications. Furthermore, not only large proteins ("proteins of interest") but also small peptides can be stably expressed by C-terminal linking to $N^{pro}$. A high expression rate forces the fusion protein into inclusion bodies. After purification, N$^{pro}$ is - according to the present invention - refolded and cleaves itself off in the tubular reactor.

It is essential that the protein of interest to be produced by the present invention is attached C-terminally after Cys168 of the N$^{pro}$ autoprotease, because this is the cleavage site where the peptidic bond between the C-terminus of the N$^{pro}$ moiety (at Cys168) and the protein of interest is cleaved in the course of step (c) according to the present invention.

Step (b), the dissolving or solubilisation step is carried out in chaotropic agents such as urea or guanidinium chloride at high concentrations in combination with reducing agents to abolish false formed disulfide bonds. Due to its simplicity refolding by dilution is widely used to initiate renaturation allowing the formation of the correct biological active structure.

The terms "kosmotrope" (order-maker) and "chaotrope" (disorder-maker) originally denoted solutes that stabilized, or destabilized respectively, proteins and membranes. Later they referred to the apparently correlating property of increasing, or decreasing respectively, the structuring of water. Such properties may vary dependent on the circumstances, method of determination or the solvation shell(s) investigated. An alternative term used for kosmotrope is "compensatory solute" as they have been found to compensate for the deleterious effects of high salt contents (which destroy the natural hydrogen bonded network of water) in osmotically stressed cells. Both the extent and strength of hydrogen bonding may be changed independently by the solute but either of these may be, and has been, used as measures of order making. It is, however, the effects on the extent of quality hydrogen bonding that is of overriding importance. The ordering effects of kosmotropes may be confused by their diffusional rotation, which creates more extensive disorganized junction zones of greater disorder with the surrounding bulk water than less hydrated chaotropes. Most kosmotropes do not cause a large scale net structuring in water.

Ionic kosmotropes (or: "antichaotropes" to distinguish them from non-ionic kosmotropes) should be treated differently from non-ionic kosmotropes, due mainly to the directed and polarized arrangements of the surrounding water molecules. Generally, ionic behaviour parallels the Hofmeister series. Large singly charged ions, with low charge density (e.g. $SCN^-$, $H_2PO_4^-$, $HSO_4^-$, $HCO_3^-$, $I^-$, $Cl^-$, $NO_3^-$, $NH_4^+$, $Cs^+$, $K^+$, $(NH_2)_3C^+$ (guanidinium) and $(CH_3)_4N^+$ (tetramethylammonium) ions; exhibiting weaker interactions with water than water with itself and thus interfering little in the hydrogen bonding of the surrounding water), are chaotropes whereas small or multiply-charged ions, with high charge density, are kosmotropes (e.g. $SO_4^{2-}$, $HPO_4^{2-}$, $Mg^{2+}$, $Ca^{2+}$, $Li^+$, $Na^+$, $H^+$, $OH^-$ and $HPO_4^{2-}$, exhibiting stronger interactions with water molecules than water with itself and therefore capable of breaking water-water hydrogen bonds). The radii of singly charged chaotropic ions are greater than 1.06Å for cations and greater than 1.78Å for anions. Thus the hydrogen bonding between water molecules is more broken in the immediate vicinity of ionic kosmotropes than ionic chaotropes. Reinforcing this conclusion, a Raman spectroscopic study of the hydrogen-bonded structure of water around the halide ions $F^-$, $Cl^-$, $Br^-$ and $I^-$ indicates that the total extent of aqueous hydrogen bonding increases with increasing ionic size and an IR study in HDO:$D_2O$ showed slow hydrogen bond reorientation around these halide ions getting slower with respect to increasing size. It is not unreasonable that a solute may strengthen some of the hydrogen bonds surrounding it (structure making; e.g. kosmotropic cations will strengthen the hydrogen bonds donated by the inner shell water molecules) whilst at the same time breaking some other hydrogen bonds (structure breaker; e.g. kosmotropic cations will weaken the hydrogen bonds accepted by the inner shell water molecules). Other factors being equal, water molecules are held more strongly by molecules with a net charge than by molecules with no net charge; as shown by the difference between zwitterionic and cationic amino acids.

Weakly hydrated ions (chaotropes, $K^+$, $Rb^+$, $Cs^+$, $Br^-$, $I^-$, guanidinium$^+$) may be "pushed" onto weakly hydrated surfaces by strong water-water interactions with the transition from strong ionic hydration to weak ionic hydration occurring where the strength of the ion-water hydration approximately equals the strength of water-water interactions in bulk solution (with $Na^+$ being borderline on the strong side and $Cl^-$ being borderline on the weak side). Neutron diffraction studies on two important chaotropes (guanidinium and thiocyanate ions) show their very poor hydration, supporting the suggestion that they preferentially interact with the protein rather than the water. In contract to the kosmotropes, there is little significant difference between the properties of ionic and nonionc chaotropes due to the low charge density of the former.

Optimum stabilization of biological macromolecule by salt requires a mixture of a kosmotropic anion with a chaotropic cation.

Chaotropes break down the hydrogen-bonded network of water, so allowing macromolecules more structural freedom and encouraging protein extension and denaturation. Kosmotropes are stabilizing solutes which increase the order of water (such as polyhydric alcohols, trehalose, trimethylamine N-oxide, glycine betaine, ectoine, proline and various other zwitterions) whereas chaotropes create weaker hydrogen bonding, decreasing the order of water, increasing its surface tension and destabilizing macromolecular structures (such as guanidinium chloride and urea at high concentrations). Recent work has shown that urea weakens both hydrogen bonding and hydrophobic interactions but glucose acts as a kosmotrope, enhancing these properties. Thus, when urea molecules are less than optimally hydrated (about 6 - 8 moles water per mole urea) urea hydrogen bonds to itself and the protein (significantly involving the peptide links) in the absence of sufficient water, so becoming more hydrophobic and hence more able to interact with further sites on the protein, leading to localized dehydration-led denaturation. Guanidinium is a planar ion that may form weak hydrogen bonds around its edge but may establish strongly-held hydrogen-bonded ion pairs to protein carboxylates, similar to commonly found quaternary structural arginine-carboxylate "salt" links. Also, guanidinium possesses rather hydrophobic surfaces that may interact with similar protein surfaces to enable protein denaturation. Both denaturants may cause protein swelling and destructuring by sliding between hydrophobic sites and consequently dragging in hydrogen-bound water to complete the denaturation.

Generally the kosmotropic/chaotropic nature of a solute is determined from the physical bulk properties of water, often at necessarily high concentration. The change in the degree of structuring may be found, for example, using NMR or vibrational spectroscopy. Protein-stabilizing solutes (kosmotropes) increase the extent of hydrogen bonding (reducing the proton and $^{17}O$ spinlattice relaxation times) whereas the NMR chemical shift may increase (showing weaker bonding e.g. the zwitterionic kosmotrope, trimethylamine N-oxide) or decrease (showing stronger bonding e.g. the polyhydroxy kosmotrope, trehalose). Trehalose shows both a reduction in chemical shift and relaxation time, as to a lesser extent does the protein stabilizer $(NH_4)_2SO_4$, whereas NaCl only shows a reduction in chemical shift and the protein destabilizer KSCN shows an increase in relaxation time and a reduction in chemical shift. Vibrational spectroscopy may make use of the near-IR wavelength near 5200 cm$^{-1}$ ($v_2 + v_3$ combination), which shifts towards longer wavelength (smaller wavenumber) when hydrogen bonds are stronger.

One of the most important kosmotropes is the non-reducing sugar $\alpha,\alpha$-trehalose. It should perhaps be noted that trehalose has a much more static structure than the reducing sugars, due to its lack of mutarotation, or the other common non-reducing disaccharide, sucrose, due to its lack of a furan ring.

Accordingly, the term "chaotropic conditions" has to be regarded individually on the nature of the liquid starting preparation (which may e.g. be a solution, a suspension, an emulsion, a two- or three phase liquid system, etc.), especially - in preparations containing more than one phase - on the aqueous phase of the preparation. Correspondence of chaotropic conditions ("corresponding to [..] urea") may be easily determined by the methods mentioned above as well as by applying the teachings of the Hofmeister series. Addition of various substances in the starting liquid has to be checked in individual cases in order to provide non-aggregating conditions. For example, the use of reduction agents should be optimised to correspond to an amount of 0.05 to 50 mM dithiothreitole (DTT), especially 0.1 to 10 mM DTT. Furthermore, also the addition of detergents may, as described above, influence the chaotropicity of the starting preparation.

The term "denatured form" (or "non-refolded form") in the meaning of the present invention designates the biologically inactive form of the expressed recombinant polypeptides, especially the fusion proteins according to the N$^{pro}$ technology, as obtained as a product of the recombinant production process, usually as obtained after solubilising the inclusion bodies (under conditions under which the native three dimensional structure of the fusion protein is disrupted).

The term "refolding" (or "renaturing") refers to the mechanism during which the solubilized protein regains its native conformation and biological activity (or the biological activity of the part, respectively), i.e. reconstituting a protein from its denatured, inactive state to its active form (see also: Kaar et al., Biotech. Bioeng. 104 (2009), 774-784). A "refolding buffer" is consequently the liquid phase wherein the polypeptides dissolved from the IBs (i.e. the recombinantly produced polypeptides, usually still in their denatured form) are allowed to refold. Usually this liquid phase is provided as a "buffer", i.e. an aqueous solution consisting of a mixture of a weak acid and its conjugate base or a weak base and its conjugate acid. Such a buffer changes its pH very little over a certain range when a small amount of strong acid or base is added to it and thus it is used to prevent changes in the pH of a solution. The refolding buffer is therefore usually used as a means of keeping pH at a nearly constant value during the refolding step. Buffers are also preferably used during other processing steps, such as steps (b), (d) or further processing steps downstream (or steps (a) or cultivation steps upstream). Such buffers may preferably also provide the necessary ionic, redox potential, conductivity, etc. conditions that are optimized for refolding for a given polypeptide.

The term "autoproteolytic function", "autoproteolytic activity" or "autoproteolytic" refers to the autoproteolytic activity of one of the moieties of the fusion protein, which is inhibited while the fusion protein is in its denatured state and which is activated upon partial (i.e. the autoprotease moiety) or complete refolding of the fusion protein. As used herein the term "autoprotease" shall refer to a polypeptide that possesses autoproteolytic activity and is capable of cleaving itself from a polypeptide moiety, i.e. the N$^{pro}$ autoprotease cleaves itself from the protein of interest part of the fusion protein according to the present invention.

As used herein, the term "recovering" shall refer to the obtaining of the protein of interest in purified form, i.e. by essentially separating the protein of interest from other proteins present in the expression/processing system, especially the autoprotease, but also all other proteins or other components which should not be present in the intermediate or final product. The protein of interest recovered by the method according to the present invention may be commercialised in the bulk form obtained directly from the cleaving step according to the present invention or further purified and/or formulated into a specific formulation, e.g. into a pharmaceutical formulation.

As used herein the term "inclusion bodies" shall refer to insoluble aggregates containing heterologous recombinant polypeptides (e.g. the fusion protein according to the N$^{pro}$ aspect of the present invention) present in the cytoplasm of transformed host cells. These appear as bright spots under the microscope and can be recovered by separation of the cytoplasm. The inclusion bodies are usually solubilised using a chaotropic agent. Upon solubilisation inclusion bodies are dissolved and a monomolecular suspension with substantially reduced intra- and inter-molecular interactions is aimed. Preferred solvents are urea, guanidine HCl and strong ionic detergents as N-lauroylsarcosine. In another embodiment of the present invention inclusion bodies are also solubilised using an aqueous alcohol solution at alkaline pH or simply an aqueous solution at alkaline pH.

As used herein the term "solubilisation" (herein synonymously used also as "dissolving" (of inclusion bodies)) shall refer to the process necessary to dissolve the inclusion bodies in step (b) according to the present invention. Solubilisation is aimed to result in a monomolecular dispersion of the polypeptides with minimum intra- and inter-molecular interactions. A preferred way of solubilisation of inclusion bodies within the scope of the present invention, is conducted by suspension in 50 mM Tris/HCl, 8 M urea, pH 7.3, with a reducing agent, e.g. 50 mM DTT, in the case that oxidized cysteine residues are present. According to another specific embodiment, solubilisation may be performed in the presence of 1-Thioglycerol, especially in the case that oxidized cysteine residues are present. Preferably 10 to 500 mM 1-Thioglycerol, more preferred 50 to 200 mM, especially about 100 mM 1-Thioglycerol are used for solubilisation. In the case that the inactive fusion protein is produced soluble within the cell, the clarified cell homogenate is subjected to the further work up described for the solubilized inclusion bodies.

Accordingly, in a preferred method the inclusion bodies were generated in a recombinant production system, preferably in a prokaryotic host cell, especially in E. coli host cells.

Such a host cell, preferably a prokaryotic host cell, especially an E. coli host cell, contains an expression vector capable of expressing the recombinant polypeptides in the host cell. The transformed (bacterial) host cell, i.e. the expression strain, may be cultivated in accordance with microbiological practice known per se. The host strain may be generally brought up starting from a single colony on a nutrient medium, but it is also possible to employ cryo-preserved cell suspensions (cell banks). The strain is generally cultivated in a multistage process in order to obtain sufficient biomass for further use.

On a small scale, this can take place in shaken flasks, it being possible in most cases to employ a complex medium (for example LB broth). However, it is also possible to use defined media (for example citrate medium). Since the expressed recombinant polypeptide is produced in the form of insoluble inclusion bodies according to the present invention, the culture will in these cases be carried out at relatively high temperature (for example 30°C or 37°C). Inducible systems are particularly suitable for producing inclusion bodies (for example with the trp, lac, tac or phoA promoter).

On a larger scale, the multistage system consists of a plurality of bioreactors (fermenters), it being preferred to employ defined nutrient media. In addition, it is possible greatly to increase biomass and product formation by metering in particular nutrients (fed batch). Otherwise, the process is analogous to the shaken flask. In the process according to the present invention, the inclusion bodies are isolated from the host cell in a manner known per se. For example, after the fermentation has taken place, the host cells are harvested by centrifugation, micro filtration, flocculation or a combination thereof, preferably by centrifugation. The wet cell mass is disintegrated by mechanical, chemical or physical means such as high pressure homogenizer, beads mills, French press, Hughes press, osmotic shock, detergents, enzymatic lysis or a combination thereof. Preferably, disruption of the cells takes place by high pressure homogenization. The recombinant polypeptide is deposited as inclusion bodies that can be obtained for example by means of high-pressure dispersion or, preferably, by a simple centrifugation at low rotor speed. The inclusion bodies are separated by centrifugation or microfiltration or a combination thereof. The purity in relation to the desired polypeptide of interest can then be improved by multiple resuspension of the inclusion bodies in various buffers, for example in the presence of NaCl (for example 0.5 1.0 M) and/or detergent (for example Triton X 100). Preferably the purity of the inclusion body preparation is improved by several washing steps with various buffers (e.g. 0.5 % Deoxycholate followed by two times 1 M NaCl solution and finally distilled water). This usually results in removal of most of the foreign polypeptides from the inclusion bodies.

Preferably, step (b) and/or (c) is performed in a buffer, especially a phosphate, hydrogen phosphate or Tris/HCl buffer. The buffer may also comprise salts, such as NaCl, polyols, such as glycerol or carbohydrates, or detergents, such as non-ionic detergents, anionic detergents, cationic detergents, zwitterionic detergents, non-detergent sulfobetains, e.g. cetyl trimethylammonium chloride (CTAC), sodium dodecyl sulfate (SDS), lithium dodecyl sulfate (LDS) or N-lauroyl sarcosine.

It is also preferred to perform step (c) in the presence of a buffer comprising NaCl, preferably of 50 to 1000 mM NaCl, especially of 100 to 500 mM NaCl; phosphate ions and/or hydrogen phosphate ions, preferably 5 to 500 mM phosphate and/or hydrogen phosphate, especially 10 to 200 mM phosphate and/or hydrogen phosphate; glycerol, preferably 1 to 10 % glycerol, especially 2 to 8 % glycerol; Tris/HCl, preferably 1 mM to 5 M Tris/HCl, especially 5 mM to 2 M Tris/HCl. The buffer used in step (c) can further contain one or more of the following ingredients: L-arginine, low concentrations of denaturants, detergents, cyclodextrins, polyethylene glycol, low-molecular weight non-detergent zwitterionic agents such as sulfobetains, substituted pyridines and pyrroles, and acid substituted aminocyclohexanes.

According to a preferred embodiment of the method according to the present invention, the inclusion bodies are suspended in water before introducing the inclusion bodies into the tubular reactor for performing the dissolving step, preferably in 10 to 90 % v/v to 90 to 10 % v/v, especially in 30% v/v to 70 % v/v to 70 % v/v to 30 % v/v.

As already stated, the dissolving (solubilisation) in step (b) is performed in chaotropic conditions corresponding to a urea concentration of more than 5 M, preferably more than 6 M, especially more than 7.5 M. If guanidinium hydrochloride is used, preferred guanidinium hydrochloride concentrations in step (c) are from 1 to 4 M, preferably 1.5 to 3.5 M, especially 2 to 3 M. Again, also combinations of different chaotropes can be applied, e.g. a mixture of urea and guanidinium hydrochloride.

Preferably, step (b) and/or step (c) is performed at a pH of 5 to 11, preferably at a pH of 6 to 9.5, especially at a pH from 6.5 to 8.5.

Step (b) may also be performed in the presence of NaOH or KOH, preferably of more than 5 mM NaOH or KOH, preferably more than 25 mM NaOH or KOH, more preferred of more than 50 mM NaOH or KOH, especially more than 100mM NaOH or KOH.

The theory and process technology for tubular reactors are well known in the art (and also referred to generically in the example section). There are a lot of process and plant options available to further optimise industrial production of the recombinant polypeptides. For example, the process components are introduced into the tubular reactor by peristaltic pumps. Examples for production architectures and set-ups are disclosed and analysed in the example section.

Preferably, (at least) steps (a) to (d) of the method according to the present invention are performed in a continuous manner, especially wherein step (c) is performed by pulse refolding (see example 2) or refolding in loops (see examples 4 and 5).

According to another aspect, the present invention relates to a method for producing a marketable preparation of recombinantly produced polypeptides, wherein a method for refolding recombinantly produced polypeptides according to the present invention as disclosed herein is performed and wherein the refolded recombinantly produced polypeptides are isolated and, optionally, further purified and finished to a marketable preparation.

The most preferred use of this method is in the field of producing recombinant proteins for pharmaceutical purposes, i.e. wherein the marketable preparation is a pharmaceutical preparation comprising the recombinantly produced polypeptide or (if, e.g. the N$^{pro}$ technology is used) a part thereof and a pharmaceutically acceptable excipient.

Examples of preferred proteins of interest with human pharmaceutical activity are cytokines such as interleukins, for example IL-6, interferons such as leukocyte interferons, for example interferon a2B, growth factors, in particular haemopoietic or wound-healing growth factors, such as G-CSF, erythropoietin, or IGF, hormones such as human growth hormone (hGH), antibodies or vaccines. Also very short polypeptides having only 5 to 30 amino acid residues can be produced as protein of interest by the present technology. The N$^{pro}$ technology has specific advantages in an expression system making use of inclusion bodies, because the strong aggregation bias of the fused autoprotease facilitates the formation of inert inclusion bodies, almost independent of the fusion partner. Accordingly, almost any protein of interest is producible with the present system in high amounts and yields. Reports are e.g. available for expression of synthetic interferon-a1; toxic gyrase inhibitor CcdB, a short 16-residue model peptide termed pep6His, human proinsulin, synthetic double domain D of staphylococcal protein A (sSpA-D$_2$), keratin-associated protein 10-4 (KRTAP10-4), synthetic green fluorescent protein variant (sGFPmut3.1), synthetic inhibitorial peptide of senescence evasion factor with N-terminal cysteine (C-sSNEVi), synthetic inhibitorial peptide of senescence evasion factor with randomized amino acid sequence with C-terminal cysteine (sSNEVscr-C); recombinant human monocyte chemoattractant protein 1 (rhMCP-1) So far, the only limitations with respect to high yields have been suspected for chaperones and proteins with comparable properties of supporting protein folding. Such proteins as fusion partners could suppress the aggregation bias of an N$^{pro}$ molecule, leading to lower yields due to less aggregation. Nevertheless, the present technology can even be applied for expressing such proteins counter-acting aggregation.

The fusion protein according to the present invention can additionally contain auxiliary sequences, such as affinity tags or refolding aid moieties; it may also contain more than one protein of interest (it can e.g. contain two or three or four or even more proteins of interest which may be separated from each other at a later stage or even at the same stage as the cleavage by the Npro autoprotease).

The present method can in principle be applied for production of any protein of interest, especially for all proteins known to be producible by the N$^{pro}$ autoprotease technique. Since the method according to the present invention is suitable for large-scale manufacturing and pharmaceutical good manufacturing practice, it is preferred to produce a protein for therapeutic use in humans with the present method, preferably a human recombinant protein or a vaccination antigen. Preferred proteins of interest therefore include the proteins referred to as the 151 recombinant pharmaceuticals approved for human use by the FDA or by the EMEA by 2009 in the review of Ferrer-Miralles et al. (Microbial Cell Factories 8 (2009), 17 doi:10.1186/1475-2859-8-17), especially the products based on proteins which have already been produced in an E. coli host cell: Dukoral (Oral cholera vaccine), Pegasys (Peginterferon alfa-2a), PegIntron (Peginterferon alfa-2b), Infergen (Interferon alfacon-1). Rebetron (Interferon alfa-2b), Roferon A (Interferon alfa-2ª), Viraferon (Interferon alfa-2b), ViraferonPeg (Peginterferon alfa-2b), Intron A (Interferon alfa-2b), Beromun (Tasonermin), Actimmune (Interferon gamma-1b), IPLEX (Mecasermin rinfabate recombinant), Kepivance (Palifermin), Neulasta (Pegfilgrastim), Neumega (Oprelvekin), Neupogen (Filgrastim), Humalog (Insulin lispro), Humatrope (Somatotropin), Humulin (Human insulin), Insuman (Human insulin), Lantus (Insulin glargine), Fortical (Salmon calcitpnin), Apidra (Insulin glulisine), Exubera (Human insulin), Forcaltonin (Salmon calcitonin), Forsteo (Teriparatide), Forsteo/Forteo (Teriparatide), Genotropin (Somatotropin), Glucagon, Increlex (Mecasermin), Insulin human Winthrop (Insulin human), Norditropin (Somatropin), Nutropin (Somatropin), NutropinAQ (Somatropin), Optisulin (Insulin glargine), Preotact (Human parathyroid hormone), Protropin (Somatrem), Somavert (Pegvisomant), Betaferon/Betaseron (Interferon beta-1b), Lucentis (Ranibizumab), Natrecor (Nesiritide), Rapilysin/Retavas e (Reteplase), Ontak (Denileukin diftitox), Kineret (Anakinra), and Omnitrope (Somatropin).

With the present invention the refolding step is specifically adapted in a tubular reactor. The method according to the present invention provides a solution to the problem of processing inclusion bodies to the active protein, where long mixing times at large scale operations is avoided. Using pumps, tubings and various tube connectors, the tubular reactor has the flexibility to be easily constructed into different configurations than other reactors, suiting different protein refolding behaviors and refolding times. The tubings high surface area to volume allows fast heat transfer, thus better temperature control. Disposable tubings can also be used, avoiding cleaning and sterilizing steps. Due to its continuous flow characteristic, it enables bioprocesses to become truly continuous, allowing improved scalability and offers possibility of a direct connection to capture systems such as expanded bed chromatography.

In general, continuous bioprocessing is shown to offer higher productivity and a smaller footprint than traditional batch bioprocessing. These continuous strategies include periodic counter-current chromatography and multicolumn countercurrent solvent gradient purification. Other advantages of continuous processes include steady-state operation, small equipment size, high volumetric productivity, streamlined process flow, low cycle times and reduced capital cost.

In the present invention, continuous refolding of proteins is performed in a tubular reactor. Different tubular reactor configurations using 3 different model proteins were setup and shown in the example section.

The first configuration is the continuous refolding of EDDIE-pep6His in a tubular reactor, using pumps and valves from a chromatography work station. The artificial peptide, pep6His will cleave from EDDIE-pep6His when refolded, where yields were previously found to be concentration independent.

The second model protein used is 6His-EDDIE-GFPmut3.1, where continuous refolding and pulse refolding of 6His-EDDIE-GFPmut3.1 were performed in a tubular reactor using peristaltic pumps. In contrast to EDDIE-pep6His, strong concentration dependence was previously found, where the $1^{st}$ order refolding process is competing with the $2^{nd}$ order aggregation process. For such competing protein refolding and aggregation process, higher refolding yields can be reached by pulse renaturation or fed batch dilution. This is possible as a low protein concentration favors the $1^{st}$ order refolding to the $2^{nd}$ order aggregation pathway. Slowly increasing the protein concentration in the process will therefore favor the refolding pathway. The third configuration is the continuous refolding of Carbonic Anhydrase II (CAII) in a tubular reactor with temperature leap tactic.

Lastly, productivity calculations at different refolding concentrations for batch and tubular protein refolding of EDDIE-pep6His and EDDIE-GFPmut3.1 refolding at industrial scale were established.

While previous alternatives focus only on the protein refolding step, current study extended and integrated this continuous strategy with other processing steps. Using EDDIE-pep6His as a model protein, a continuous dissolution, refolding and precipitation tubular reactor was established. This simplified system allows 3 process steps to be performed in one continuous flow, eliminating any holding times. Because IBs are more stable at cooled temperatures, it can be fed at slow rates into tubular reactor inlets for dissolution, reducing tubular reactor volume and cost. Small equipment size brings about greater flexibility and mobility. While keeping residence times constant, flow rates, tube diameter, tube lengths can be changed easily suiting different process conditions. If disposable materials are used for tubular reactor, less cleaning is needed with lower upfront investment cost.

This continuous process was initially performed where refolding and acid precipitation of pre-dissolved EDDIE-pep6His is continuously performed in a tubular reactor over 3 and 21 hours. Later, an additional dissolution step was also included and performed over 5 and 20 hours. The refolding kinetic studies at the start and end of process were compared and pep6His yields were collected from tubular reactor outlets over process time. For each process run, different tubular dimensions, flow rates and refolding protein concentrations were performed, showing the robustness of the tubular system. Refolding and pep6His yields obtained in batch reactor were also similar to tubular reactor in similar conditions. To establish complete process purification, pep6His after acid precipitation was further purified and concentrated using reverse-phased chromatography and subsequently lyophilized. The final quality of pep6His was further verified with mass spectroscopy.

The present invention is further described by the following examples and the drawing figures, yet without being restricted thereto.

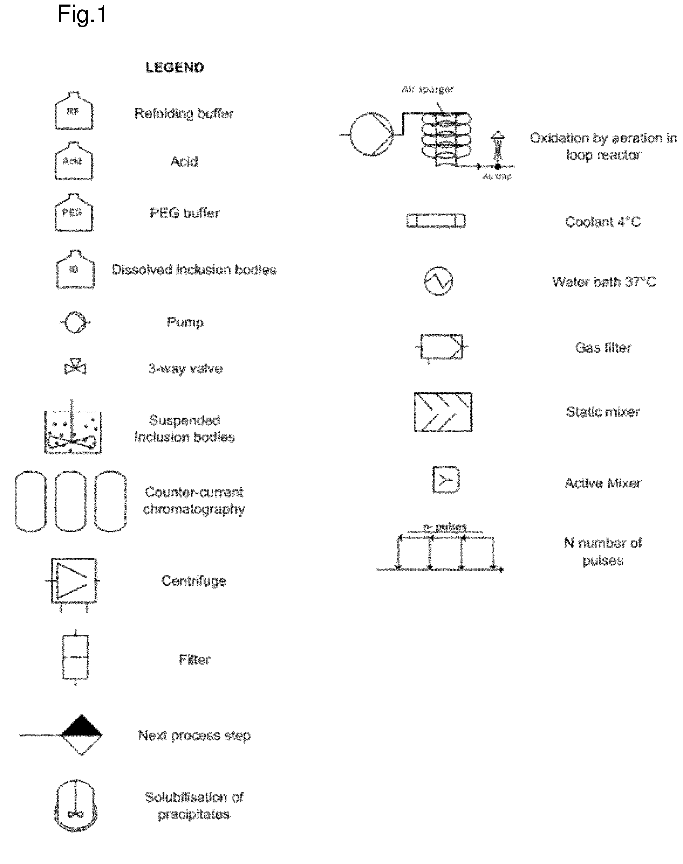

Figure 1: Figure Legends.
Figure 2: Continuous IBs dissolution, refolding, acid precipitation, centrifugation, filtration and counter-current chromatography of EDDIE-pep6His.

Figure 3: (A) Refolding kinetic pathways in batch and tubular reactor (B) Recovery yield of pep6His after acid precipitation in batch reactor and tubular reactor over 20 hours. No visible decrease observed in pep6His recovery over process time. (C) Mass spectroscopy analysis showed the authentic pep6His at 1.839kDa after RP-chromatography.

Figure 4: SDS-PAGE analysis of EDDIE-pep6His in different sections of the tubular reactor. Top band is uncleaved EDDIE-pep6His while lower band is cleaved EDDIE.

Figure 5: Continuous pulse refolding, selective PEG precipitation and centrifugation of EDDIE-GFP

Figure 6: (A) Refolding kinetics of 6His-EDDIE-GFPmut3.1 in batch and tubular reactors using refolding and pulse refolding strategies. Final refolding concentration of 6His-EDDIE-GFPmut3.1 in all refolding methods was 0.2mg/ml at 0.9M urea. (B) Dotted symbols show refolded sample yields collected in fraction collectors from tubular reactor outlets every 12.3 minutes over 19.3 hours to show steady state has reached. Lines represent refolded yields from batch reactors. Samples were taken at refolding equilibrium after 48 hours.

Figure 7: Continuous temperature leap tactic refolding, selective PEG precipitation and centrifugation of precipitated native carbonic anhydrase II.

Figure 8: Refolding yields of 5mg/ml CAII between refolding at 20°C and temperature leap tactic from 4°C for 50 minutes and immediately to 37°C for 20 minutes.

Figure 9: Direct continuous refolding from IBs, oxidation in loop reactor, filtration and counter-current chromatography for recombinant human vascular endothelial growth factor (rhVEGF).

Figure 10: Oxidation kinetic yields of recombinant human vascular endothelial growth factor (rhVEGF) by HPLC analysis over time as shown in literature study by (Pizarro et al. 2009).

Figure 11: This continuous process strategy is suitable for proteins which require long refolding times and also an oxidation step to form disulphide bonds such as EDDIE-proinsulin. With this plan the whole purification of the protein can be performed, from this solution of IBs to final product clarification.

Figure 12: PID of continuous dissolution, loop refolding, PEG/acid precipitation for buffer exchange in solubilizing step, pulse addition of dissolved precipitate, oxidation by aeration in loop reactor and acid precipitation by selective removal of impurities of EDDIE-sProinsulin.

EXAMPLES:

**Continuous processing of proteins using an integrated tubular reactor**

Table 1 shows a few continuous process strategies for the patent application of the various tubular reactor concepts. Different strategies are required to exploit the different proteins with different refolding behaviors. The main concepts are:

1. Continuous inclusion bodies (IBs) dissolution, refolding and precipitation
2. Continuous pulse refolding and precipitation
3. Continuous temperature leap tactic refolding and precipitation
4. Continuous refolding and oxidation in loop reactor
5. Continuous IBs dissolution, refolding in loop reactor, precipitation, solubilisation and oxidation in loop reactor.

Table I: Proteins with different properties and refolding behaviors are as shown. Different tubular reactor constructions are needed for different model proteins.

| Model protein | Approximated refolding speed | Other refolding behavior | Tubular reactor type | PID |
|---|---|---|---|---|
| EDDIE-pep6His | Fast (3h) | Yield is concentration independent (Achmüller et al. 2007), requires removal of EDDIE after cleavage | Integrated tubular reactor with refolding and acid precipitation | Figure 2 |
| EDDIE-GFP | Medium (8h) | Yield is concentration dependent (Achmüller et al. 2007), requires removal of EDDIE after cleavage | Integrated tubular reactor with pulse refolding and PEG precipitation | Figure 5 |

(continued)

| Model protein | Approximated refolding speed | Other refolding behavior | Tubular reactor type | PID |
|---|---|---|---|---|
| Carbonic Anhydrase II | Fast (1h) | Yield is higher when refolded at 4°C and then at 37°C. Requires temperature leap tactic. (Xie and Wetlaufer 1996) | Tubular reactor with cooling and heating element for temperature leap tactic refolding | Figure 7 |
| Vascular endothelial growth factor (rhVEGF) | Fast (30min), excluding oxidation | Requires oxidation step (Pizarro et al. 2009) | Integrated tubular reactor with refolding and loop reactor for oxidation | Figure 9 |
| EDDIE-sProinsulin | Slow (16h) Slow (72h) (Achmüller et al. 2007) | Requires buffer exchange and oxidation step | Integrated loop reactor for refolding, PEG precipitation for buffer exchange and loop reactor for oxidation | Figure 12 |

Glossary

    **Active mixer:** Mixing element for homogenous mixing of converging process solutions by agitation of an element fitted into the mixer chamber.

    **Concentration dependent refolding:** Protein refolding behavior where its final yield decreases with increasing refolding concentration. (Kaar et al. 2009)

    **Concentration independent refolding:** Protein refolding behavior where its final yield does not depend on refolding concentration. (Kaar et al. 2009)

    **Counter-current chromatography:** Continuous chromatography whereby first column is loaded to saturation and the breakthrough is loaded onto the second column. The saturated column is washed and eluted with the same methods used in batch chromatography. (Jungbauer 2013)

    **Loop reactor:** A tubular reactor arranged in circuit, where process fluid is circulated within the reactor until desired residence time is completed. This enables continuous processes to be performed in long lasting reaction steps while avoiding long tubular reactors.

    **N number of loops:** The scaling out of the loop reactors according to the process volume. Gives process more flexibility in order to adapt to different process volumes.

    **Pulse refolding:** A feeding strategy by adding denatured protein into refolding buffer in discrete amounts over a specific time interval to increase refolding yields for concentration dependent refolding.

    **Selective precipitation:** Process to precipitate out only the targeted protein from a protein solution.

    **Static mixer:** Mixing element for homogenous mixing of converging process solutions by inducing disruptive flow within the element.

    **Temperature leap tactic refolding:** A refolding method for carbonic anhydrase II by refolding initially at 4°C to suppress aggregation and reach stable intermediate before refolding at 37°C to reach native state. (Xie and Wetlaufer 1996)

**1. Continuous dissolution, refolding and acid precipitation of EDDIE-pep6His in tubular reactor from inclusion bodies**

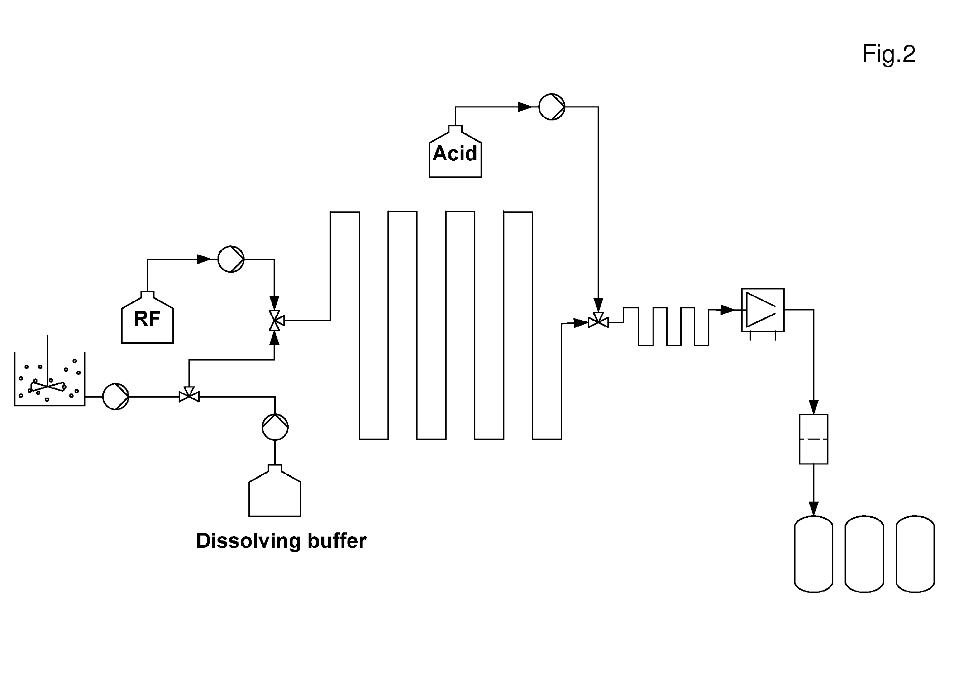

EDDIE-pep6His is a fusion protein expressed in E.Coli as inclusion bodies. Figure 2 shows a continuous strategy suitable to purify the target peptide pep6His. First, suspended IBs and dissolving buffer are continuously pumped and mixed into the dissolution section where dissolution of IBs can take place. Dissolved IBs will then be continuously transported and mixed with refolding buffer (RF) for refolding to occur in the refolding section. After refolding, pep6His will be cleaved from the EDDIE-pep6His fusion protein. Subsequently, acid will be mixed with refolded solution so that EDDIE and any uncleaved fusion proteins will be precipitated out of the solution, while pep6His will stay in the solution. From the precipitation outlet, a continuous centrifuge will then separate the precipitates from the supernatant. This supernatant will then be filtered and further purified by counter-current chromatography.

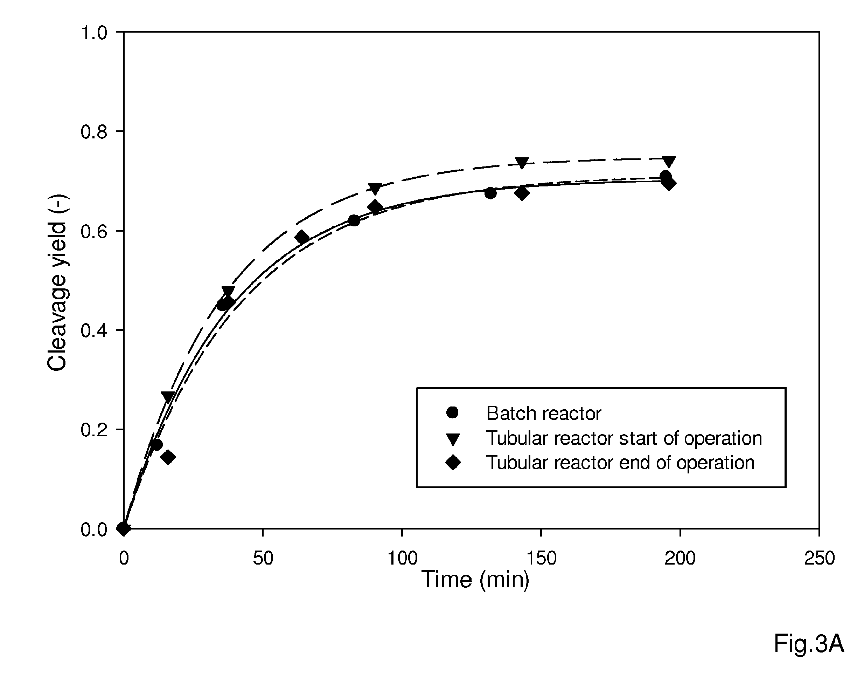

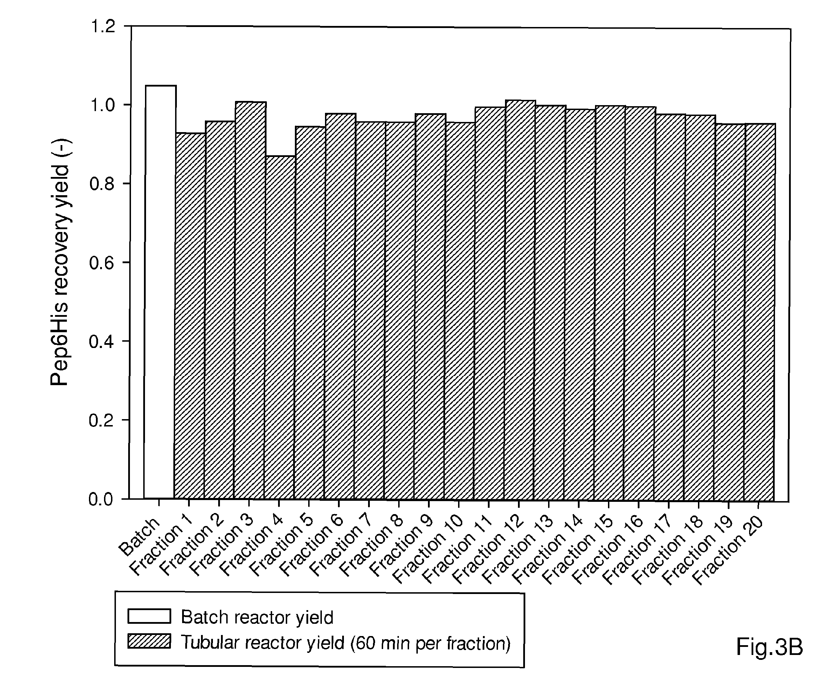

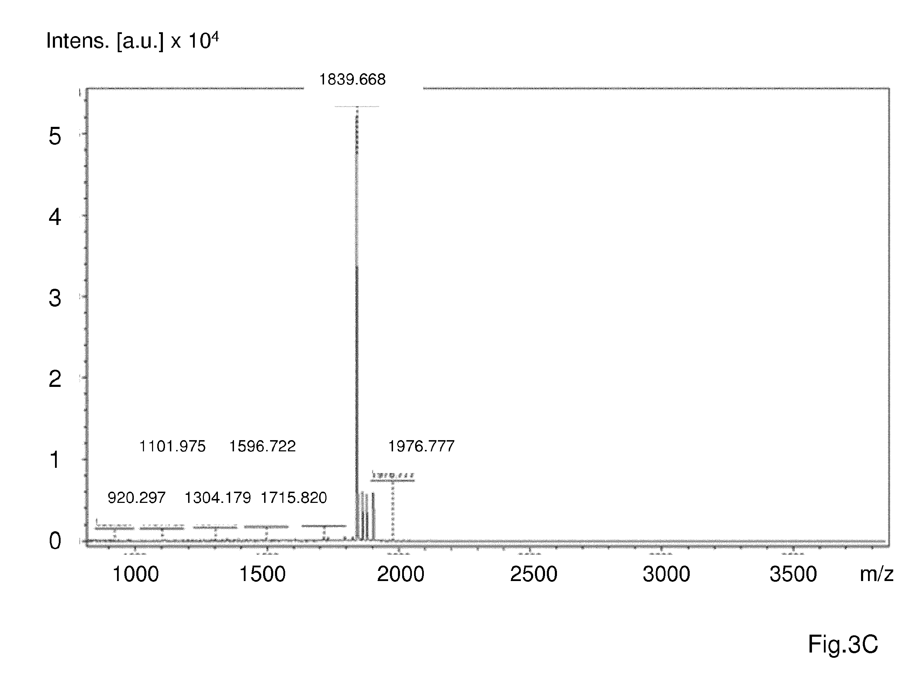

An integrated process in the tubular reactor from dissolution, refolding and precipitation step was experimentally performed, along with a batch process with similar conditions for comparison. The dimensions and operating parameters of the tubular reactor are shown in Table II, while the experimental results in the IB dissolution, refolding and precipitation section are shown in Figure 3.

Table II: Tubular reactor dimensions and operating values of EDDIE-pep6His processing from dissolution to precipitation.

|  |  |  |
|---|---|---|
| Dissolution | Inner diameter (mm) | 1.5 |
|  | Tube lenght (m) | 0.3 |
|  | Tubular volume (ml) | 0.5 |
|  | \Volumetric flow (ml/min) | 0.1.52 |
|  | Residence time (min) | 3.5 |
| Refolding | Refolding concentration (mg/ml) | 0.7 |
|  | Inner diameter (mm) | 3.2 |
|  | Tube length (m) | 37.1 |
|  | Tubular volume (ml) | 298.4 |
|  | Volumetric flow (ml/min) | 1.52 |
|  | Residence time (min) | 195 |
| Precipitation | Inner diameter (mm) | 3.2 |
|  | Tube length (m) | 1 |
|  | Tubular volume (ml) | 8.0 |
|  | Volumetric flow (ml/min) | 1.68 |
|  | Residence time (min) | 4.8 |
| Total process time (h) |  | 20 |

Cleavage kinetics quantified by HPLC analysis at the start and the end of process after 20 hours were relatively similar in the refolding section. In addition, pep6His recovery yields were quite consistent throughout process time in steady state over 20 hours as seen in Table III, while the rate constants for $k_1$ and $k_2$ calculated as shown in Table III were in the same order of magnitude between the batch and the tubular reactor.

Table III: Refolding kinetic constants and yields in tubular and batch reactor of EDDIE-pep6His.

|  | Kinetic constants and yield | Results |
|---|---|---|
| Batch reactor | Refolding constant $k_1$ ($s^{-1}$) | $3.1 \times 10^{-4}$ |
|  | Misfolding constant $k_2$($s^{-1}$) | $1.3 \times 10^{-4}$ |
|  | Refolding yield (-) | 71 |
| Tubular reactor | Refolding constant start $k_1$ ($s^{-1}$) | $3.4 \times 10^{-4}$ |
|  | Misfolding constant start $k_2$ ($s^{-1}$) | $1.2 \times 10^{-4}$ |
|  | Starting yield (-) | 74 |
|  | Refolding constant end $k_1$ ($s^{-1}$) | $2.9 \times 10^{-4}$ |
|  | Misfolding constant end $k_2$ ($s^{-1}$) | $1.2 \times 10^{-4}$ |
|  | Ending yield (-) | 70 |

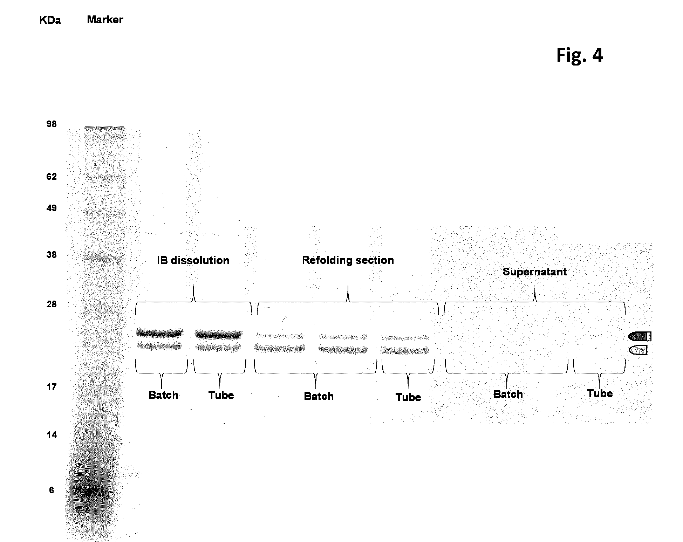

These show that the tubular reactor was able to reach steady state throughout the 20 hour process. Furthermore, the authentic peptide was obtained in this process as confirmed by mass spectroscopy. Additional analysis by SDS-PAGE analysis in Figure 4 was shown to be good agreement with HPLC analysis.

Productivity calculations of EDDIE-pep6His in Table IV are quantitatively compared between the batch and tubular reactor in the dissolution, refolding and precipitation steps.

Table IV: Productivity calculations of EDDIE-pep6His in tubular and batch reactor.

| Steps | Tubular reactor | | Batch reactor | |
|---|---|---|---|---|
| Dissolution | Inner diameter (mm) | 1.50 | Filling flow (ml/min) | 50.00 |
| | Tube lenght (m) | 0.30 | Dissolution volume (ml) | 180.00 |
| | Tubular volume (ml) | 0.50 | Dissolution reactor volume (ml) | 225.00 |
| | Volumetric flow (ml/min) | 0.15 | Total filling time (min) | 3.60 |
| | Residence time (min) | 3.50 | Dissolution time (min) | 1.5.00 |
| Refolding | Inner diameter (mm) | 3.20 | Filling flow (ml/min) | 50.00 |
| | Tube length (m) | 37.10 | Refolding volume (ml) | 1800 |
| | Tubular volume (ml) | 298.40 | Total filling time (min) | 36.00 |
| | Volumetric flow (ml/min) | 1.52 | Refolding time (min) | 195.00 |
| | Residence time (min) | 195.00 | | |
| Precipitation | Inner diameter (mm) | 3.20 | Filling/emptying flow (ml/min) | 50.00 |
| | Tube length (m) | 1.00 | Precipitant volume (ml) | 216 |
| | Tubular volume (ml) | 8.00 | Precipitation volume (ml) | 2016 |
| | Volumetric flow (ml/min) | 1.68 | Reactor volume (ml) | 2520 |
| | Residence time (min) | 4.80 | Total filling time (min) | 4.32 |
| | | | Precipitation time (min) | 30.00 |
| | | | Emptying time (min) | 40.32 |
| Process summary | Final concentration (mg/ml) | 0.63 | Final concentration (mg/ml) | 0.63 |
| | Total process time (h) | 20.00 | Total process time (h) | 5.15 |
| | Total processed volume (ml) | 2016 | Total processed volume (ml) | 2016 |
| | Total reactor volume (ml) | 306.9 | Total reactor volume (ml) | 2745 |
| | Approximate refolded yield (-) | 0.70 | Approximate refolded yield (-) | 0.70 |
| | Total refolded amount (mg) | 882 | Total refolded amount (mg) | 882 |
| | **Productivity (mg/L/h)** | **143.70** | **Productivity (mg/L/h)** | **62.34** |

Tubular reactor dimensions and operating values were derived from experimental conditions shown in Table II. Average yields were obtained from experimental results shown in Table III.

To ensure a fair comparison, the amount of volume processed in the tubular reactor over 20 hours must be the same as the total amount that can be processed in a batch reactor. Therefore the batch reactor volume is scaled up to match the desired total refolded amount.

The general productivity equation is calculated by the refolded amount, divided by the reactor volume and process time.

$$productivity = \frac{Total.refolded.Amt}{(reactor\_volume)(process\_time)} \qquad \textbf{Equation 1}$$

While the total refolded amount in batch and tubular reactor is the same, because the process conditions such as refolding buffer and protein concentration are similar, reactor volume and process time is different.

Reactor volume in tubular reactor is calculated as:

$$tubular\_reactor\_volume = (volumetric\_flow\_rate).(residence\_time) \qquad \textbf{Equation 2}$$

Reactor volume in batch reactor is calculated as:

$$batch\_reactor\_volume = (dilution\_factor).(protein\_volume).(1.25) \qquad \textbf{Equation 3}$$

Multiplying by 1.25 is to ensure there is sufficient space for the working volume within the reactor volume.

Process time from Equation 1 in tubular reactor is the time required to feed process solution into the tubular reactor at steady state. In the present calculations, this will be 20 hours. On the contrary, process time in the batch reactor is the filling time plus the reaction time of each process step. An additional emptying time is also required at the final precipitation step. The filling and emptying times will depend on the pump flow into the batch reactor. In the present case this is 50ml/min. Specifically, two batch reactors will be required in the process. The 1st reactor is for the dissolution step while the 2nd reactor is for the refolding and precipitation step.

As seen from calculations in Table IV, productivity in a continuous process is higher at 143.7mg/L/h to 62.34mg/L/h in batch process. While the process time in continuous process is higher, the total reactor volume is significantly lower, resulting in an overall higher productivity in the continuous process. In addition, other benefits in continuous process are listed as follows in Table V.

Table V: Benefits of a continuous system using a tubular reactor.

| | Tubular reactor | Batch reactor |
|---|---|---|
| Full integration between process steps | ✓ | × |
| Steady state reached | ✓ | × |
| Full utilization of reactors | ✓ | × |
| Less moving parts | ✓ | × |
| Holding times eliminated | ✓ | × |
| No protein degradation from holdups | ✓ | × |
| Continuous loading to chromatography | ✓ | × |
| Smaller equipment | ✓ | × |
| Higher productivity | ✓ | × |
| Higher reproducibility in scale up | ✓ | × |
| Better process flexibility | ✓ | × |
| Better equipment mobility | ✓ | × |

## 2. Continuous pulse refolding and precipitation in tubular reactor from dissolved EDDIE-GFP

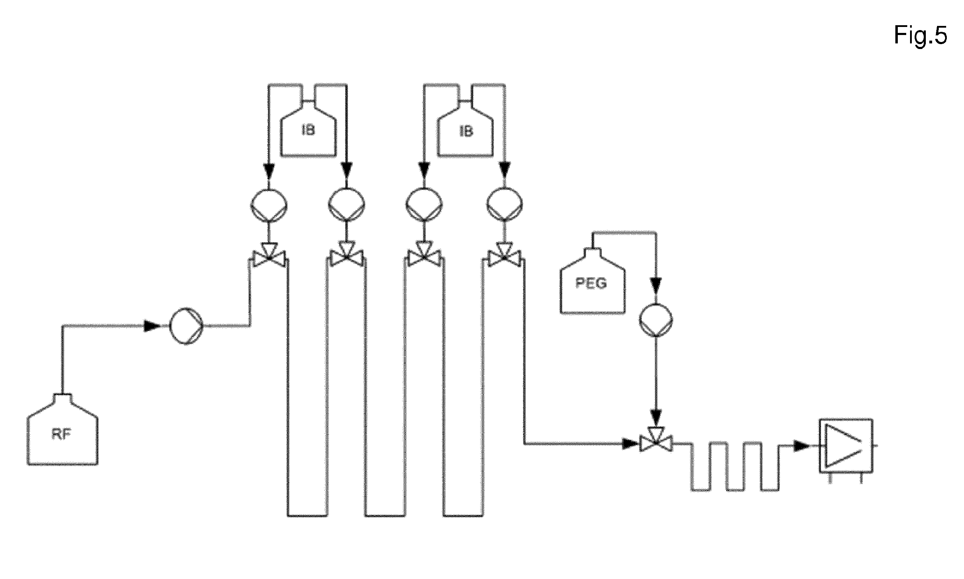

Figure 5 shows the continuous process to purify native GFP from dissolved EDDIE-GFP inclusion bodies. Pulse refolding in a tubular reactor allows higher yields than standard refolding in addition to a continuous process. The cleavage and refolding yield of GFP from EDDIE-GFP is a concentration dependent process, due to competition between the 1st order refolding and 2nd order aggregation process. In order for refolding yields to be higher, the aggregation process can be suppressed by refolding at low protein concentrations to allow higher proportions of protein to go to the native pathway. Using pulse refolding, aggregation is suppressed by incrementally increasing the refolding concentration in intervals to the desired concentration, as oppose to refolding to the correct concentration in one addition. When pulse refolding is completed in the tubular reactor, GFP will be cleaved from EDDIE and refolded to its native form. Subsequently, selective PEG precipitation can be performed to precipitate out the native GFP and separated by continuous centrifugation.

Experiment is performed to show yields can be increased by pulsed refolding in a tubular reactor and steady state can be reached. Refolding and pulse refolding in the tubular reactor of denatured EDDIE-GFP from inclusion bodies were experimentally performed, along with refolding and pulse refolding in batch at similar conditions for comparison. The dimensions and operating parameters of the tubular reactor are shown in Table VI, while the experimental yield results and derived kinetic constants were shown in Table VII.

Table VI: Tubular reactor dimensions and operating values of EDDIE-GFP refolding and pulse refolding.

| Experiment | Refolding | Pulse refolding |
|---|---|---|
| Denatured protein concentration (mg/ml) | 2 | 2 |
| Urea concentration before refolding (M) | 9 | 9 |
| Final refolding concentration (mg/ml) | 0.2 | 0.2 |
| Residual denaturant concentration (M) | 0.9 | 0.9 |
| Tube inner diameter (mm) | 3.2 | 3.2 |
| Tube length (m) | 36.5 | 35.1 |
| Pump flow for denatured protein ($\mu$l/min) | 122 | 30.6 x 4 |

(continued)

| Experiment | Refolding | Pulse refolding |
|---|---|---|
| Pump flow for refolding buffer ($\mu$l/min) | 1.100 | 1100 |
| Pulse at specific residence time (min) | 0 | 0,60,120,180 |
| Total residence time (min) | 240 | 240 |

Table VII: Refolding kinetic constants and yields in tubular and batch reactor of EDDIE-GFP.

| | Kinetic constants and yield | Refolding | Pulse refolding |
|---|---|---|---|
| Batch reactor | Refolding constant $k_1$ ($s^{-1}$) | $3.23 \times 10^{-5}$ | - |
| | Aggregation constant $K_2$ ($M^{-1}s^{-1}$) | 39.6 | - |
| | Refolding yield (-) | 35.4 | 44.4 |
| Tubular reactor | Refolding constant start $k_1$ ($s^{-1}$) | $3.26 \times 10^{-5}$ | - |
| | Misfolding constant start $K_2$ ($M^{-1}s^{-1}$) | 43.5 | - |
| | Refolding yield (-) | 33.4 | 41.7 |

Due to laboratory and material limitations, the tubular reactors residence time were 4 hours long, sufficient to perform pulse refolding instead of the full refolding length of 48 hours. This is sufficient to proof the benefits of pulse refolding. Furthermore, the refolding kinetics and at equilibrium yield were continuously analyzed after collection from the tubular reactor outlet in fraction collectors.

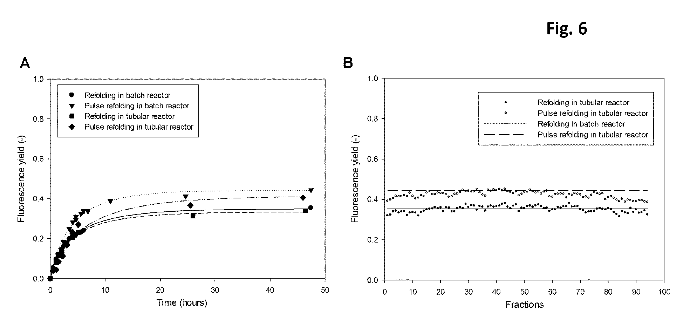

Results for a continuous strategy of EDDIE-GFP, are shown in Figure 6. Refolding yields were also able to reach steady state for both refolding strategies for more than 19 hours in the tubular reactor. From Table VII, pulse refolding strategy showed a 25% higher refolding yield as compared to standard refolding in both batch and tubular reactor. Additionally, refolding and aggregation constants in standard refolding were at the same order of magnitude between batch and tubular reactor.

For a fair comparison, residence time in the tubular reactor is increased to 48 hours to accommodate the whole refolding step of EDDIE-GFP. Values of yields, process volumes, process time at steady state performed derived from performed experiment were used in calculations. Equation 1, Equation 2 and Equation 3 were used to calculate productivity. Total processed volume of batch and tubular were kept the same for correct comparison. As summarized in Table VIII, results show that productivity of EDDIE-GFP refolding is 25% higher in pulse refolding strategies as compared to standard refolding in the respective batch and tubular reactor. In addition, productivity is 20% higher in in tubular reactor than batch reactor in the respective refolding strategy.

Table VIII: Productivity calculations of EDDIE-GFP for standard and pulse refolding in batch and tubular reactor

| Tubular reactor | Refolding | Pulse refolding | Batch reactor | Refolding | Pulse refolding |
|---|---|---|---|---|---|
| Inner diameter (mm) | 3.2 | 3.2 | Filling/emptying flow (ml/min) | 50 | 50 |
| Volumetric flow (ml/min) | 1.22 | 1.22 | Denatured protein volume (ml) | 139 | 139 |
| Residence time (h) | 48 | 48 | Refolding buffer volume (ml) | 1254 | 1254 |
| Tube length (m) | 437.6 | 437.6 | Refolding volume (ml) | 1393 | 1393 |
| Tubular volume (ml) | 3519 | 3519 | Reactor volume (ml) | 1741 | 1741 |
| | | | Total filling/empty time(min) | 27.9 | 27.9 |
| | | | Refolding time (h) | 48 | 48 |
| Final concentration (mg/ml) | 0.2 | 0.2 | Final concentration (mg/ml) | 0.2 | 0.2 |
| | | | Total process time (h) | 48.9 | 48.9 |
| Total process time (h) | 19 | 19 | | | |

(continued)

| Tubular reactor | Refolding | Pulse refolding | Batch reactor | Refolding | Pulse refolding |
|---|---|---|---|---|---|
| Total processed volume (ml) | 1393 | 1393 | Total processed volume (ml) | 1393 | 1393 |
| Total reactor volume (ml) | 3519 | 3519 | Total reactor volume (ml) | 1741 | 1741 |
| Approximate refolded yield (-) | 33.4 | 41.7 | Approximate refolded yield (-) | 35.4 | 44.4 |
| Total refolded amount (mg) | 93.1 | 116.2 | Total refolded amount (mg) | 98.6 | 123.7 |
| **Productivity (mg/L/h)** | **1.39** | **1.74** | **(mg/L/h)** | **1.16** | **1.45** |

**3. Continuous temperature leap tactic refolding and precipitation in tubular reactor of carbonic anhydrase II**

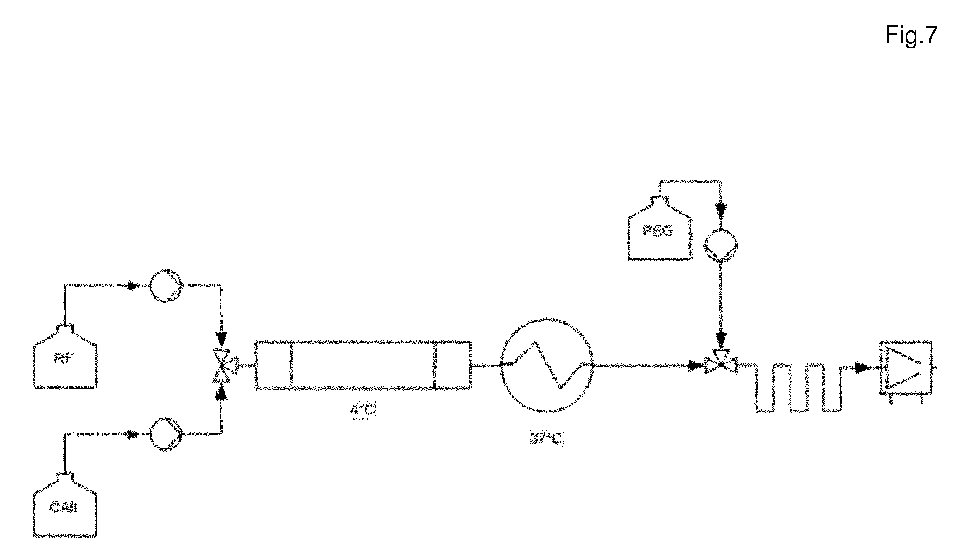

Figure 7 shows a strategy to refold proteins which require specific temperatures. The tubular reactor allows faster heat transfer than batch reactor, especially at large scale. Using a temperature leap tactic from 4°C to 37°C, carbonic anhydrase II (CAII) can be refolded at a higher yield as compared to refolding at just 20°C. After refolding, the native CAII could be selective precipitated by PEG before being separated by continuous centrifuge.

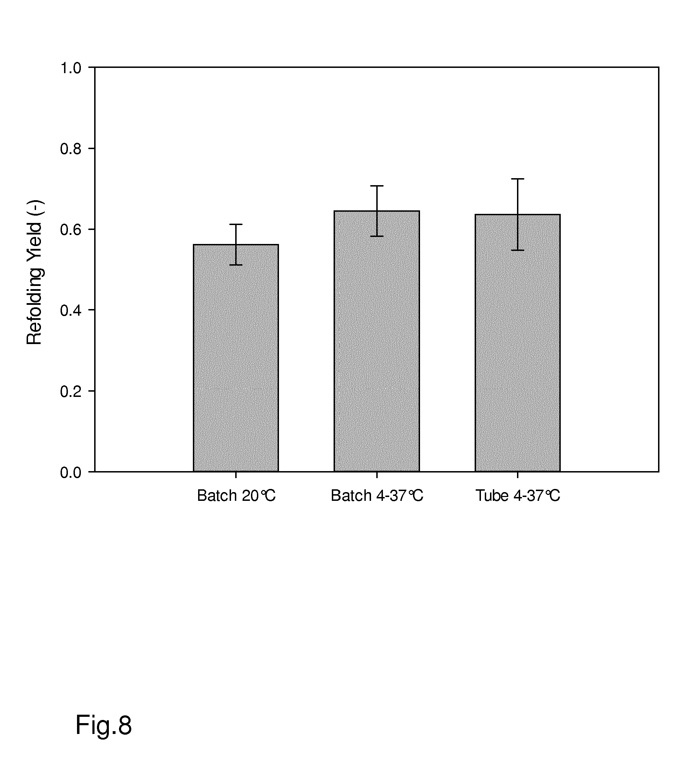

Results for continuous strategy of carbonic anhydrase II in Figure 7 in the refolding section by pulse and standard refolding is shown in Figure 8. Using temperature leap tactic, higher refolding yields can be obtained as compared to refolding at 20°C.

**4. Continuous refolding and oxidation by aeration in tubular reactor of recombinant human vascular endothelial growth factor**

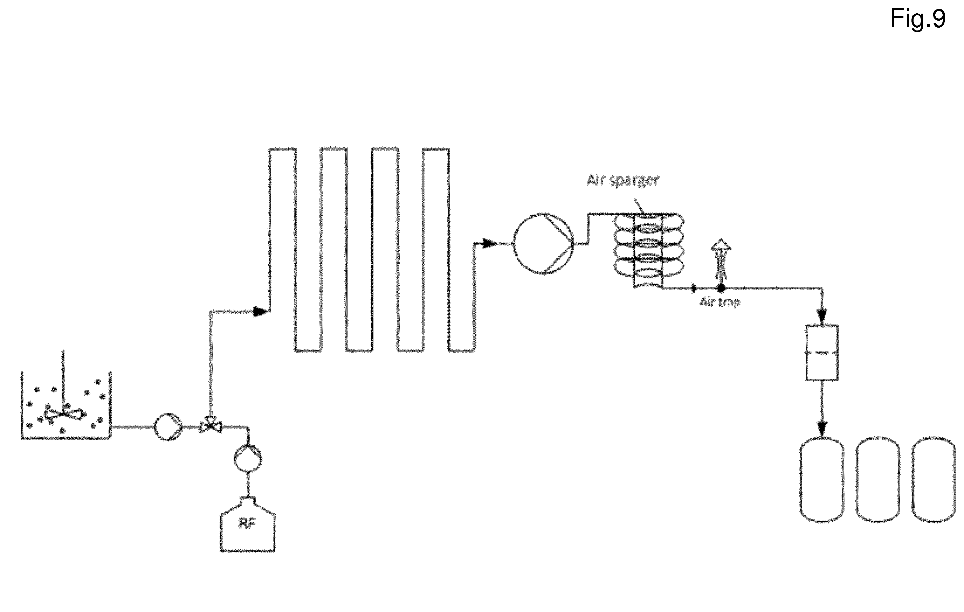

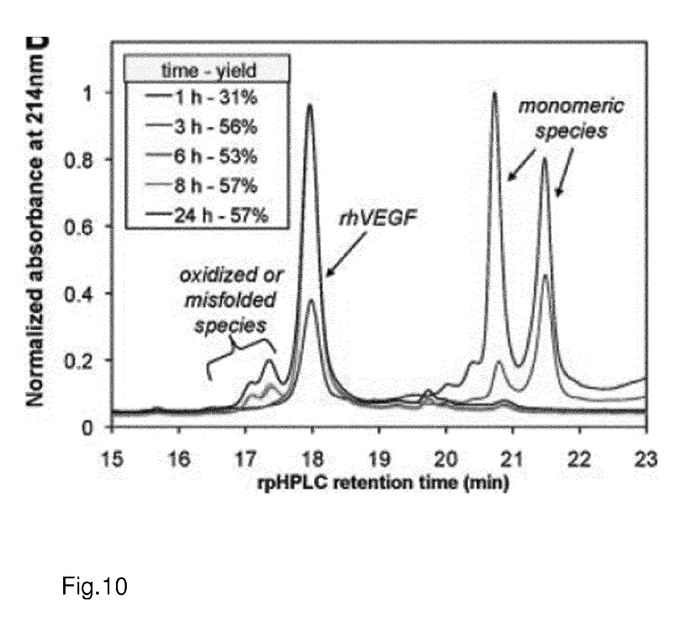

Oxidation results of recombinant human vascular endothelial growth factor (rhVEGF) are shown in Figure 9. Suspended IBs can be directly refolded. Subsequently aeration can be performed to form disulphide bonds of refolded rhVEGF. Air can be removed via the air trap before folded and oxidized rhVEGF will undergo filtration and continuous chromatography. Results performed by (Pizarro et al. 2009) in batch refolding and oxidation in Figure 10 showed that the native and oxidized form of rhVEGF could be formed by direct IB refolding and oxidation by aeration. Likewise, this process steps could be adopted into a continuous process as shown in Figure 9.

**5. Continuous IBs dissolution, refolding in loop reactor, precipitation, solubilisation and oxidation in loop reactor**

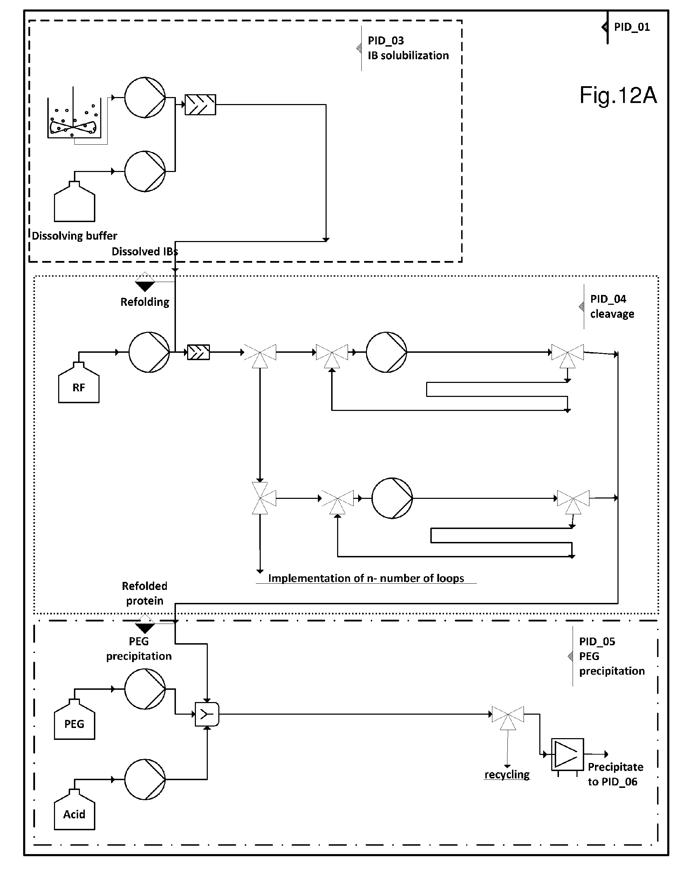

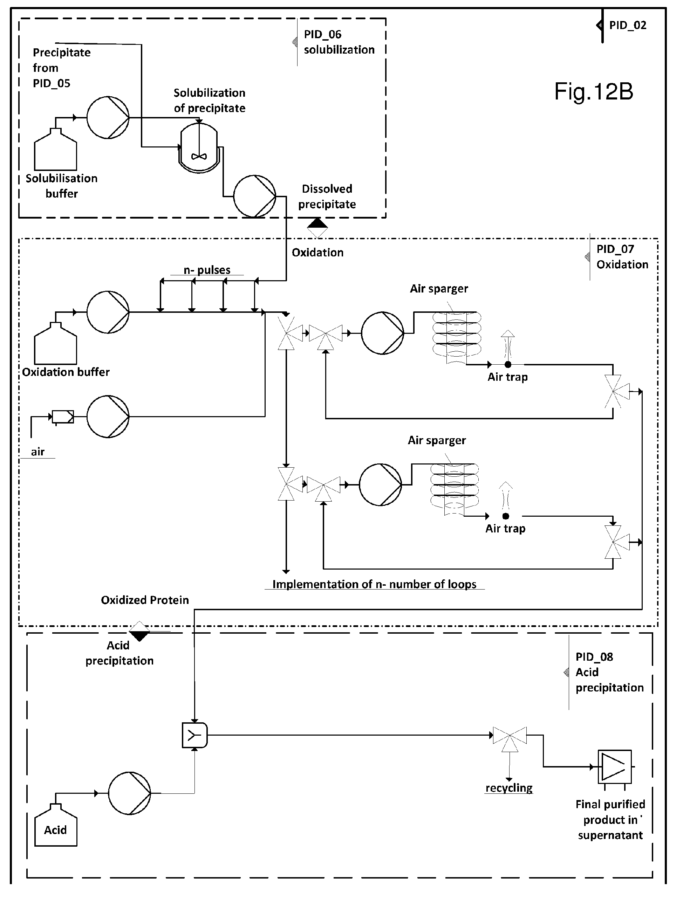

The process strategy to refold proteins with long refolding times and require oxidation as shown in Figure 12 could be applied to EDDIE-sProinsulin found in literature (Achmüller et al. 2007). The cleavage properties of proinsulin is very long at 72h as shown in Table IX.

Table IX: Different refolding cleavage yields and times of EDDIE-pep6His and EDDIE-proinsulin as shown in (Achmüller et al. 2007). Refolding time for EDDIE-proinsulin is much higher at 72 hours and oxidation to form disulphide bonds is required after cleavage due to the presence of 6 cysteines.

| Target proteins and peptides[a] | N terminus | pI[b] | Number of cysteines | Number of amino acids | MW[b] (kDa) | Initial concentration of fusion protein ($\mu$g/ml) | cleavage time | N[pro] cleavage rate (%) | | EDDIE cleavage rate (%) | |
|---|---|---|---|---|---|---|---|---|---|---|---|
| | | | | | | | | *In vivo* | *In vitro* | *In vivo* | *In vitro* |
| pep6His | SVDKL | 6.55 | 0 | 16 | 1.8 | 10-20 | 40 min | 20[c] | 60[d] | 5[c] | 80[d] |
| sProinsutin | FVNQH | 5.20 | 6 | 86 | 9.4 | 10-20 | 72 h | 0[c] | 1[d] | 0[c] | 20[d] |

[a]Proteins derived from codon-optimized genes are by preceded by an 's'. [b]Estimated with Expasy ProtParam tool (http://ca.expasy.org/tools/protparam.html). [c]Determined by densitometry of SDS-PAGE gels. [d]Determined by densitometry of SDS-PAGE gels; in vivo cleavage was subtracted.

In this example, adopting the tubular reactor in looping arrangement has the advantages of a standard tubular reactor such as shorter mixing times, faster heat transfer and continuous operation. Additionally, other advantages include:

1) Flexible scale: Operation in small and big scale viable by implementation of different number of loops. In small scale even processes with finite fluid volumes can be operated in a tubular reactor by avoiding excessive reactor lengths. As the scale and the processing volume increase only the respective number of reactor loops have to be added to the system.

2) Facilitated up scaling: Mixing and thus creating homogeneous condition and aeration is facilitated in tubular reactor. For this reason the problems of poor mixing and slow oxygen transfer in large scale can be overcome or alleviated.

3) Easy adjustment to different processing conditions: Different refolding behaviors and times of different proteins can be met by adding or eliminating reactor loops. In general different reaction kinetics can be handled by this strategy.

4) Improved monitoring of process conditions: Since, a monitoring system is implemented in every reactor loop a certain portion of the whole processing fluid is constantly monitored and thus allows to relate the monitored signal to this specific fluid. E.g. In the case of monitoring protein oxidation by measuring the redox potential it might be possible to infer the end of reaction and consequently when the fluid circulating in the loop can proceed to the next processing step.

5) Better intervention in the process: In the case of site specific breakdowns or short term local process instabilities only a small portion of the fluid (e.g. fluid which circulated in one loop) has to be discharged. Furthermore, locally shifts of monitored signals can be easier managed. If this shift lies in an acceptable range the desired process condition can be reestablished by addition of e.g. acid/base/buffer to the corresponding loop.

6) Improved flexibility and productivity of the whole production plant: Production need not be totally shut down for maintenance or cleaning. While one loop is cleaned or maintained, production can be carried out in the other loops.

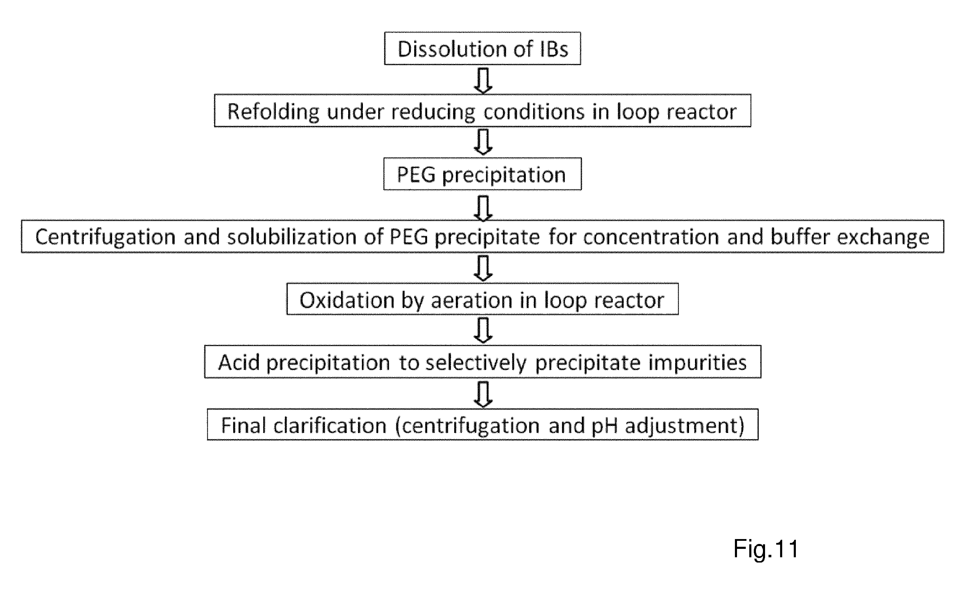

Figure 11 shows the specific steps involved in this process while Figure 12 shows a detailed PID on how this process could be adopted as a continuous strategy. Details include 1) process consists of continuous dissolution of IBs, 2) static and active mixers are implemented for better mixing, 3) the number of loops will depend on reaction time and volume of loop reactor. 4) Pulse addition is performed before oxidation by aeration process, 5) air trap to remove any air bubbles so solution can be monitored by any integrated online sensors put in place, before solution is circulated back for repeated aeration. Subsequent acid precipitation will precipitate out any impurities before precipitated impurities are continuously removed by continuous centrifugation.

References

Achmüller C, Kaar W, Ahrer K, Wechner P, Hahn R, Werther F, Schmidinger H, Cserjan-Puschmann M, Clementschitsch F, Striedner G and others. 2007. Npro fusion technology to produce proteins with authentic N termini in E. coli. Nature Methods 4(12):1037-1043.

Jungbauer A. 2013. Continuous downstream processing of biopharmaceuticals. Trends in Biotechnology 31(8):479-492.

Kaar W, Ahrer K, Dürauer A, Greinstetter S, Sprinzl W, Wechner P, Clementschitsch F, Bayer K, Achmüller C, Auer B and others. 2009. Refolding of Npro fusion proteins. Biotechnol Bioeng 104(4):774-84.

Pizarro SA, Dinges R, Adams R, Sanchez A, Winter C. 2009. Biomanufacturing process analytical technology (PAT) application for downstream processing: Using dissolved oxygen as an indicator of product quality for a protein refolding reaction. Biotechnology and Bioengineering 104(2):340-351.

Xie Y, Wetlaufer DB. 1996. Control of aggregation in protein refolding: the temperature-leap tactic. Protein Sci 5(3):517-23.

## Claims

1. Method for refolding recombinantly produced polypeptides comprising the steps of

(a) providing inclusion bodies comprising recombinantly produced polypeptides comprising the recombinantly produced polypeptides,

(b) dissolving the inclusion bodies under chaotropic conditions in so as to obtain denatured recombinantly produced polypeptides, and

(c) refolding the denatured recombinantly produced polypeptides by addition of a refolding buffer, so as to obtain refolded recombinantly produced polypeptides, and

(d) further processing the refolded recombinantly produced polypeptides by at least one processing step, wherein at least steps (c) and (d) are performed in a tubular reactor in a continuous manner.

2. Method according to claim 1 wherein step (c) is performed with changing conditions, especially with changing temperature, with changing ion concentration in the refolding buffer, with changing pH in the refolding buffer, changing protein concentration, or combinations or sequential application of such changing conditions.

3. Method according to claims 1 or 2, wherein the refolded recombinantly produced polypeptides are in step (d) subjected to a filtration, a crystallisation, a centrifugation, a precipitation, especially a precipitation wherein the recombinantly produced polypeptides are preferably obtained in the precipitate, a (continuous) chromatography, or a modification, wherein the recombinantly produced polypeptide is chemically or biologically, preferably enzymatically, modified, especially by autocleavage; a flocculation, a heat treatment, a pH treatment, or combinations thereof.

4. Method according to any one of claims 1 to 3, wherein the recombinantly produced polypeptides are fusion polypeptides comprising a polypeptide of interest and an $N^{pro}$ autoprotease.

5. Method according to any one of claims 1 to 4, wherein the inclusion bodies are suspended in water before introducing the inclusion bodies into the tubular reactor for performing the dissolving step, preferably in 10 to 90 % v/v to 90 to 10 % v/v, especially in 30% v/v to 70 % v/v to 70 % v/v to 30 % v/v.

6. Method according to any one of claims 1 to 5, wherein the the dissolving in step (b) is performed in chaotropic conditions corresponding to a urea concentration of more than 5 M, preferably more than 6 M, especially more than 7.5 M.

7. Method according to any one of claims 1 to 6, wherein step (b) and/or step (c) is performed at a pH of 5 to 11, preferably at a pH of 6 to 9.5, especially at a pH from 6.5 to 8.5.

8. Method according to any one of claims 1 to 7, wherein step (b) is performed in the presence of NaOH or KOH, preferably of more than 5 mM NaOH or KOH, preferably more than 25 mM NaOH or KOH, more preferred of more than 50 mM NaOH or KOH, especially more than 100mM NaOH or KOH.

9. Method according to any one of claims 1 to 8, wherein process components are introduced into the tubular reactor by peristaltic pumps.

10. Method according to any one of claims 1 to 9, wherein steps (a) to (d) are performed in a continuous manner, especially wherein step (c) is performed by pulse refolding or refolding in loops.

11. Method for producing a marketable preparation of recombinantly produced polypeptides, wherein a method according to anyone of claims 1 to 10 is performed and wherein the refolded recombinantly produced polypeptides are isolated and, optionally, further purified and finished to a marketable preparation.

12. Method according to claim 11, wherein the marketable preparation is a pharmaceutical preparation comprising the recombinantly produced polypeptide or a part thereof and a pharmaceutically acceptable excipient.

# Fig.1

LEGEND

| | |
|---|---|
| RF | Refolding buffer |
| Acid | Acid |
| PEG | PEG buffer |
| IB | Dissolved inclusion bodies |
| | Pump |
| | 3-way valve |
| | Suspended Inclusion bodies |
| | Counter-current chromatography |
| | Centrifuge |
| | Filter |
| | Next process step |
| | Solubilisation of precipitates |

| | |
|---|---|
| Air sparger / Air trap | Oxidation by aeration in loop reactor |
| | Coolant 4°C |
| | Water bath 37°C |
| | Gas filter |
| | Static mixer |
| | Active Mixer |
| n- pulses | N number of pulses |

Fig.2

Acid

RF

Dissolving buffer

EP 2 889 304 A1

Fig.3A

Fig.3B

EP 2 889 304 A1

Intens. [a.u.] x $10^4$

1839.668

5 ⊣

4 ⊣

3 ⊣

2 ⊣

1 ⊣          1101.975     1596.722              1976.777

   920.297    1304.179  1715.820

0 ⊣

       1000        1500        2000        2500        3000        3500      m/z

Fig.3C

**Fig. 4**

KDa   Marker

98
62
49
38   IB dissolution      Refolding section
28                                        Supernatant
     Batch   Tube      Batch      Tube      Batch      Tube
17
14
6

Fig.5

**Fig. 6**

Fig.7

EP 2 889 304 A1

Fig.8

Fig.9

Air sparger

Air trap

RF

EP 2 889 304 A1

Fig.10

EP 2 889 304 A1

```
                        ┌─────────────────────────┐
                        │     Dissolution of IBs   │
                        └─────────────────────────┘
                                    ⇩
        ┌──────────────────────────────────────────────────────┐
        │  Refolding under reducing conditions in loop reactor  │
        └──────────────────────────────────────────────────────┘
                                    ⇩
                        ┌─────────────────────────┐
                        │     PEG precipitation    │
                        └─────────────────────────┘
                                    ⇩
┌────────────────────────────────────────────────────────────────────────────────────┐
│ Centrifugation and solubilization of PEG precipitate for concentration and buffer exchange │
└────────────────────────────────────────────────────────────────────────────────────┘
                                    ⇩
            ┌──────────────────────────────────────────────┐
            │     Oxidation by aeration in loop reactor     │
            └──────────────────────────────────────────────┘
                                    ⇩
        ┌──────────────────────────────────────────────────────┐
        │  Acid precipitation to selectively precipitate impurities │
        └──────────────────────────────────────────────────────┘
                                    ⇩
        ┌──────────────────────────────────────────────────────┐
        │  Final clarification (centrifugation and pH adjustment) │
        └──────────────────────────────────────────────────────┘
```

Fig.11

Fig.12A

Fig.12B

Europäisches
Patentamt

European
Patent Office

Office européen
des brevets

# EUROPEAN SEARCH REPORT

Application Number

EP 13 19 9710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | MASAAKI TERASHIMA ET AL: "Effective refolding of fully reduced lysozyme with a flow-type reactor", PROCESS BIOCHEMISTRY, vol. 31, no. 4, 1 May 1996 (1996-05-01), pages 341-345, XP55111836, ISSN: 1359-5113, DOI: 10.1016/0032-9592(95)00069-0 * the whole document * | 1-12 | INV. C07K1/113 |
| Y,D | ALOIS JUNGBAUER: "Continuous downstream processing of biopharmaceuticals", TRENDS IN BIOTECHNOLOGY, vol. 31, no. 8, 1 August 2013 (2013-08-01) , pages 479-492, XP055111517, ISSN: 0167-7799, DOI: 10.1016/j.tibtech.2013.05.011 * the whole document * | 1-12 | |
| Y,D | KATOH S ET AL: "Continuous refolding of lysozyme with fed-batch addition of denatured protein solution", PROCESS BIOCHEMISTRY, XX, XX, vol. 35, 1 June 2000 (2000-06-01), pages 1119-1124, XP002224937, DOI: 10.1016/S0032-9592(00)00145-X * the whole document * | 1-12 | |

TECHNICAL FIELDS
SEARCHED (IPC)

C07K

-/--

The present search report has been drawn up for all claims

| Place of search | Date of completion of the search | Examiner |
|---|---|---|
| Munich | 3 April 2014 | Luo, Xinmei |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 13 19 9710

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | NOHARA D ET AL: "High performance in protease refolding by application of a continuous system using immobilized inhibitor", JOURNAL OF BIOSCIENCE AND BIOENGINEERING, ELSEVIER, AMSTERDAM, NL, vol. 98, no. 6, 1 January 2004 (2004-01-01), pages 482-486, XP004727095, ISSN: 1389-1723 * the whole document * | 1-12 | |
| Y | SCHLEGL R ET AL: "Refolding of proteins in a CSTR", CHEMICAL ENGINEERING SCIENCE, OXFORD, GB, vol. 60, no. 21, 1 November 2005 (2005-11-01), pages 5770-5780, XP027646487, ISSN: 0009-2509 [retrieved on 2005-11-01] * the whole document * | 1-12 | |
| A,D | WALTRAUD KAAR ET AL: "Refolding of N pro fusion proteins", BIOTECHNOLOGY AND BIOENGINEERING, vol. 104, no. 4, 1 November 2009 (2009-11-01), pages n/a-n/a, XP055050976, ISSN: 0006-3592, DOI: 10.1002/bit.22432 * the whole document * | 1-12 | |

TECHNICAL FIELDS
SEARCHED (IPC)

-/--

The present search report has been drawn up for all claims

| Place of search | Date of completion of the search | Examiner |
|---|---|---|
| Munich | 3 April 2014 | Luo, Xinmei |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

Europäisches
Patentamt

European
Patent Office

Office européen
des brevets

## EUROPEAN SEARCH REPORT

Application Number

EP 13 19 9710

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | YANSHENG XIE ET AL: "Control of aggregation in protein refolding: The temperature-leap tactic", PROTEIN SCIENCE, vol. 5, no. 3, 1 March 1996 (1996-03-01), pages 517-523, XP055111291, ISSN: 0961-8368, DOI: 10.1002/pro.5560050314 * the whole document * | 1-12 | |
| A | US 2007/238860 A1 (SCHLEGL ROBERT [AT]) 11 October 2007 (2007-10-11) * the whole document * | 1-12 | |

TECHNICAL FIELDS
SEARCHED (IPC)

The present search report has been drawn up for all claims

| Place of search | Date of completion of the search | Examiner |
|---|---|---|
| Munich | 3 April 2014 | Luo, Xinmei |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                     EP 13 19 9710

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2014

| Patent document cited in search report | Publication date | Patent family member(s) | Publication date |
|---|---|---|---|
| US 2007238860 A1 | 11-10-2007 | EP 1845103 A1<br>US 2007238860 A1 | 17-10-2007<br>11-10-2007 |

EPO FORM P0459

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0111057 A
- WO 0111056 A
- WO 2006113957 A
- WO 2006113958 A
- WO 2006113959 A
- EP 12198006 A
- EP 12198007 A

**Non-patent literature cited in the description**

- **ACHMÜLLER et al.** *Nature Methods,* 2007, vol. 4 (12), 1037-1043
- **JUNGBAUER et al.** *J Biotechnol,* 2007, vol. 128 (3), 587-96
- **SCHLEGL et al.** *Science,* 2005, vol. 60 (21), 5770-5780
- **TERASHIMA et al.** *Process Biochemistry,* 1996, vol. 31 (4), 341-345
- **KATOH et al.** *Process Biochemistry,* 2000, vol. 35 (10), 1119-1124
- **MULLER-SPATH et al.** *Biotechnol Bioeng,* 2010, vol. 107 (6), 974-84
- **ACHMÜLLER et al.** *Nat. Methods,* 2007, vol. 4, 1037-1043
- **ACHMÜLLER et al.** *Nat. Meth.,* 2007, vol. 4, 1037-1043
- **KAAR et al.** *Biotech. Bioeng.,* 2009, vol. 104, 774-784
- **ACHMÜLLER C ; KAAR W ; AHRER K ; WECHNER P ; HAHN R ; WERTHER F ; SCHMIDINGER H ; CSERJAN-PUSCHMANN M ; CLEMENTS-CHITSCH F ; STRIEDNER G.** Npro fusion technology to produce proteins with authentic N termini in E. coli. *Nature Methods,* 2007, vol. 4 (12), 1037-1043
- **JUNGBAUER A.** Continuous downstream processing of biopharmaceuticals. *Trends in Biotechnology,* 2013, vol. 31 (8), 479-492
- **KAAR W ; AHRER K ; DÜRAUER A ; GREINSTETTER S ; SPRINZL W ; WECHNER P ; CLEMENTS-CHITSCH F ; BAYER K ; ACHMÜLLER C ; AUER B.** Refolding of Npro fusion proteins. *Biotechnol Bioeng,* 2009, vol. 104 (4), 774-84
- **PIZARRO SA ; DINGES R ; ADAMS R ; SANCHEZ A ; WINTER C.** Biomanufacturing process analytical technology (PAT) application for downstream processing: Using dissolved oxygen as an indicator of product quality for a protein refolding reaction. *Biotechnology and Bioengineering,* 2009, vol. 104 (2), 340-351
- **XIE Y ; WETLAUFER DB.** Control of aggregation in protein refolding: the temperature-leap tactic. *Protein Sci,* 1996, vol. 5 (3), 517-23